# FASCICULE DE BREVET EUROPEEN

(11) **EP 0 631 626 B1**
(45) Date de publication et mention de la délivrance du brevet: **26.05.2004**
(21) Numéro de dépôt: 93918753.0
(22) Date de dépôt: 12.03.1993
(51) Int. Cl.: C12N 15/74, C12N 1/21

(54) **PLASMIDE THERMOSENSIBLE**
THERMOSENSITIVE PLASMIDE
THERMOSENSITIVE PLASMID

(30) Priorité: 13.03.1992 FR 9203034
(43) Date de publication de la demande: 04.01.1995
(73) Titulaire: INSTITUT NATIONAL DE LA RECHERCHE AGRONOMIQUE, 75007 Paris (FR)
(72) Inventeur: GRUSS, Alexandra, F-75003 Paris (FR); MAGUIN, Emmanuelle, F-92120 Montrouge (FR)
(74) Mandataire: Warcoin, Jacques
(86) Numéro de dépôt international: PCT/FR1993/000248
(87) Numéro de publication internationale: WO 1993/018164

(56) Documents cités:
- EP-A- 0 182 562
- EP-A- 0 243 856
- EP-A- 0 445 385
- APPLIED AND ENVIRONMENTAL MICROBIOLOGY vol. 57, no. 2, 1991, WASHINGTON D C pages 539 - 548 FEIRTAG J M;PETZEL J P;PASALODOS E;BALDWIN K A;MCKAY L L 'THERMOSENSITIVE PLASMID REPLICATION TEMPERATURE-SENSITIVE HOST GROWTH AND CHROMOSOMAL PLASMID INTEGRATION CONFERRED BY LACTOCOCCUS-LACTIS-SSP-CREMORIS LACTOSE PLASMIDS IN LACTOCOCCUS-LACTIS-SSP-LACTIS'
- JOURNAL OF BACTERIOLOGY vol. 172, no. 8, 1990, BALTIMORE US pages 4543 - 4548 SOZHAMANNAN S;DABERT P;MORETTO V;EHRLICH S D;GRUSS A 'PLUS-ORIGIN MAPPING OF SINGLE-STRANDED DNA PLASMID P-E-194 AND NICK SITE HOMOLOGIES WITH OTHER PLASMIDS' cité dans la demande
- BIOLOGICAL ABSTRACTS vol. 87 Philadelphia, PA, US; abstract no. 047423, ALONSO J C;STIEGE C A;TAILOR R H;VIRET J-F 'FUNCTIONAL ANALYSIS OF THE DNA-TS MUTANTS OF BACILLUS-SUBTILIS PLASMID PUB110 REPLICATION AS A MODEL SYSTEM'
- BIOLOGICAL ABSTRACTS vol. 92 Philadelphia, PA, US; abstract no. 075453, LEENHOUTS K J;KOK J;VENEMA G 'REPLACEMENT RECOMBINATION IN LACTOCOCCUS-LACTIS'
- BIOLOGICAL ABSTRACTS vol. 88 Philadelphia, PA, US; abstract no. 107483, PRIEBE S D;LACKS S A 'REGION OF THE STREPTOCOCCAL PLASMID PMV158 REQUIRED FOR CONJUGATIVE MOBILIZATION'
- JOURNAL OF BACTERIOLOGY vol. 174, no. 17, 1992, BALTIMORE US pages 5633 - 5638 MAGUIN E;DUWAT P;HEGE T;EHRLICH D;GRUSS A 'NEW THERMOSENSITIVE PLASMID FOR GRAM-POSITIVE BACTERIA'

## Description

La présente invention concerne un plasmide utilisable pour la modification génétique des bactéries à coloration gram positive, en particulier des bactéries lactiques présentant un intérêt industriel ou médical.

Elle concerne également des bactéries contenant un tel plasmide. Elle concerne enfin des procédés de modifications génétiques mettant en oeuvre un tel plasmide, soit pour inactiver un gène normalement présent dans le chromosome bactérien, soit pour introduire et exprimer un gène d'intérêt.

De nombreuses bactéries gram à coloration gram positive sont des sujets d'étude comme modèle biologique (par exemple les bactéries du genre Bacillus), comme souche de fermentation d'intérêt industriel (bactéries à acide lactique) ou comme pathogène (par exemple Clostridia. Listeria, Staphylococcus, Streptococcus). Beaucoup de ces souches sont caractérisées d'un point de vue physiologique mais peu ont été étudiées ou modifiées génétiquement. L'étude ou la modification des souches peut être facilitée par l'utilisation de vecteurs permettant des insertions dirigées ou non-spécifiques dans le chromosome bactérien. Des systèmes de délivrance qui sont fondés sur des vecteurs non réplicatifs sont limités aux bactéries qui peuvent être transformées avec une haute fréquence et ceux utilisant des réplicons actifs uniquement sous certaines conditions sont souvent limités à leur spectre d'hôte. Aussi la construction de souches recombinantes requiert un effort important et ne peut être appliquée avec efficacité qu'à certains microorganismes spécifiques.

L'addition, la perte ou la modification de gènes peuvent transformer le rôle d'un organisme dans un processus industriel tel que la fermentation.

La biotechnologie cherche à faciliter l'usage industriel de microorganismes. Par exemple, les bactéries lactiques sont utilisées en agroalimentaire, majoritairement pour la fabrication de produits laitiers fermentés. mais aussi en dehors de la fillière lait pour la fabrication de vin, cidre, charcuterie et ensilage.

Il est donc particulièrement souhaitable de disposer de moyens efficaces d'introduire ou de modifier spécifiquement et définitivement certains gènes dans ces organismes.

Actuellement, la modification du chromosome chez les bactéries lactiques est effectuée par l'intermédiaire d'un système par transformation d'un plasmide non réplicatif. Dans une seule étape, il est nécessaire d'avoir deux évènements de basse fréquence, la transformation par un plasmide, et une recombinaison dans le chromosome. La probabilité d'obtenir ces deux évènements dans une seule étape est le produit des probabilités de chacun ; donc une chance très faible d'obtenir la modification.

Le plasmide pWV01 est un plasmide cryptique initialement isolé chez Lactococcus lactis subsp. cremoris ; il s'agit d'un plasmide à large spécificité d'hôte, réplicatif à la fois dans des bactéries gram positif et gram négatif, notament chez E. coli, Bacillus subtilis, Lactococcus lactis, Streptococcus et Lactobacillus. Il a été caractérisé et sa séquence nucléotidique a été publiée par Leenhouts et al (1991).

Dans la demande WO 85/03495, de larges fragments de ce plasmide sont utilisés pour construire un plasmide recombinant pGK12, marqué par le gène de résistance à l'érythromycine, et/ou le gène de résistance au chloramphénicol (la chloramphénicol-acétyl-transférase (CAT)). Ce plasmide pGK12 n'est pas utilisable pour faire des intégrations dans le chromosome bactérien.

Les plasmides non réplicatifs utilisés jusqu'à présent permettent de pallier ce problème, mais ce système requiert des taux de transformation élevés pour permettre la détection d'évènements de basse fréquence tels que la transposition ou la recombinaison dans le chromosome ; or la plupart des bactéries lactiques sont faiblement transformables.

L'ensemble de ces difficultés pourrait être surmonté grâce à l'obtention d'un réplicon thermosensible, utilisable comme vecteur de livraison dans les bactéries, lactiques ou autres.

Les plasmides pE194 et PSH71 ont été décrits comme naturellement thermosensibles, au-dessus d'une température de 51° C (J. Bacteriol., 1990, 172, 4543-4548)

C'est pourquoi la présente invention a pour objet un plasmide vecteur bactérien du type comportant une origine de réplication efficace dans les bactéries gram+, caractérisé en ce qu'il comporte au moins :
- un gène marqueur qui s'exprime dans une souche hôte bactérienne,
- un système de réplication efficace qui est thermosensible (Ts) à partir d'une température compatible avec la viabilité de la souche hôte,
et en ce que la température d'inhibition de réplication est inférieure ou égale à environ 37° C.

Le fait que le plasmide selon l'invention soit non réplicatif à 37° C le rend particulièrement approprié dans le cas où les bactéries ont une température de croissance relativement basse, ou lorsqu'un choc thermique important n'est pas souhaitable. Le plasmide selon l'invention est utilisable seul, il n'a pas à être associé à un autre plasmide. L'inhibition de la réplication par des températures supérieures à environ 37° C, n'est pas souche dépendante. Il possède un large spectre d'hôtes et peut s'établir notamment chez les souches classiques appartenant au groupe comprenant : Bacillus, Enterococcus, Lactobacillus, Lactococcus, Streptococcus, Listeria, Pediococcus, Staphylococcus, Clostridia, Leuconostoc, E. coli. Parmi celles-ci, on peut citer à titre d'exemple les espèces suivantes : B. subtilis, E. faecalis, L. fermentum, L. helveticus, L. bulgaricus, L. lactis. S. pyogenes, S. thermophilus, S. sanguis, L. monocytogenes.

Le plasmide selon l'invention porte au moins un gène codant pour un marqueur de sélection, ainsi que les éléments nécessaires à son expression tels que promoteur, site de fixation des ribosomes, terminateur, etc. Des gènes de sélection sont par exemple des gènes de résistance aux antibiotiques (Erythromycine, chloramphénicol), ou permettant la croissance sur un milieu dépourvu de certains éléments, etc.

Le gène marqueur est intégré dans le chromosome en cas de recombinaison.

Par système de réplication, on entend un système comprenant une origine de réplication ainsi que la protéine induisant son fonctionnement ; ladite protéine est inactivée au-dessus d'une température inhibant le système de réplication.

Un tel plasmide se réplique normalement à 28° C, chez un grand nombre de bactéries. A une température supérieure à environ 35° C, la réplication de ce plasmide est inhibée ; cette température inhibitrice de la réplication du plasmide est relativement basse et permet la multiplication et la croissance normale de la plupart des bactéries, en particulier des bactéries lactiques. La température recommandée pour l'inactivation efficace de ce plasmide est de 37° C.

Selon l'un de ses aspects, la présente invention a pour objet un plasmide vecteur, caractérisé en ce qu'il contient le plus grand fragment Cla 1 du plasmide pWV01, présentant au moins une mutation dans la région Thal-Rsal.

Plus particulièrement, un plasmide vecteur à réplication thermosensible selon l'invention présente au moins une mutation dans la région correspondant à RepA du plasmide pWV01. La protéine RepA est codée par l'un des 4 cadres ouverts de lecture (ORF) identifiés sur pWV01, l'ORF A, et est nécessaire pour la réplication.

Des mutations préférées de ce plasmide se trouvent dans les positions 972, 977, 980 et 987 de la séquence nucléotidique de pWV01.

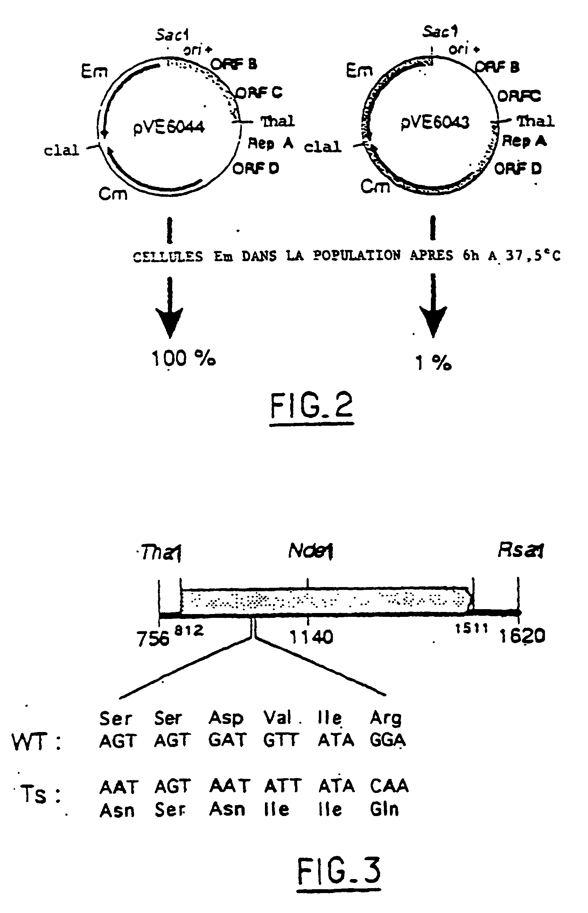

La protéine RepA codée par le plasmide selon l'invention présente, par rapport au type sauvage, les modifications représentées à la figure 3, à savoir le remplacement de :
- Ser par Asn,
- Asp par Asn,
- Val par Ile,
- Arg par Gln.

Un tel plasmide constitue un vecteur suicide à large spécificité d'hôte, d'un type unique jusqu'à nos jours dans le domaine des bactéries lactiques.

En effet, il permet de dissocier en deux étapes, l'intégration dans le chromosome. Dans la première étape de transformation, le plasmide est établi dans la cellule. Dans la deuxième étape, l'événement d'intégration dans le chromosome est sélectionné par élévation de la température. On peut ainsi modifier génétiquement des bactéries réputées peu transformables.

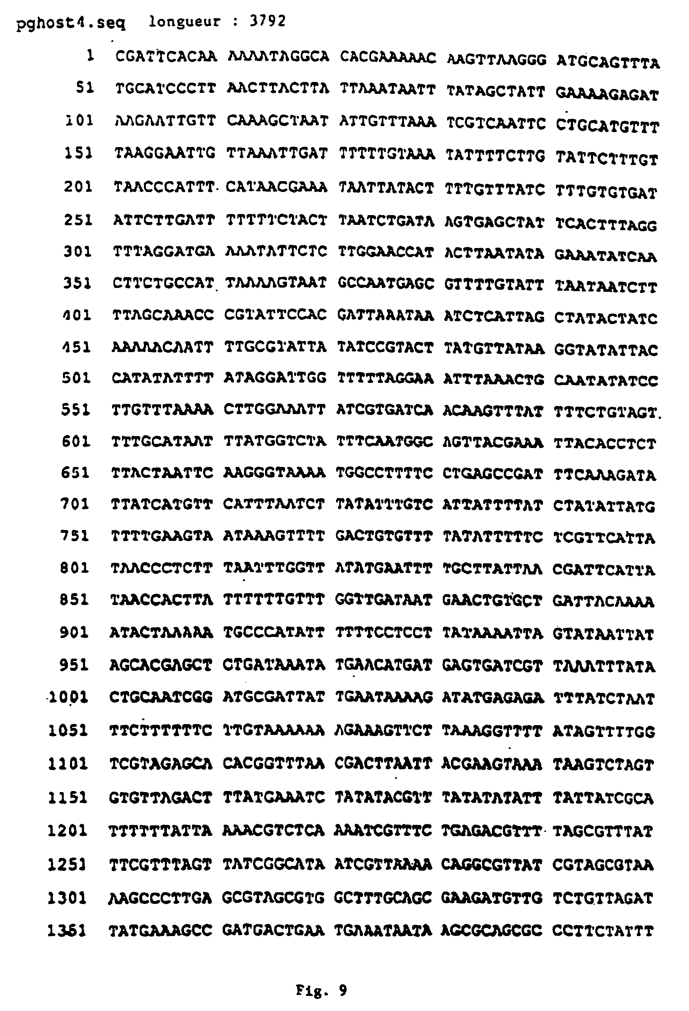

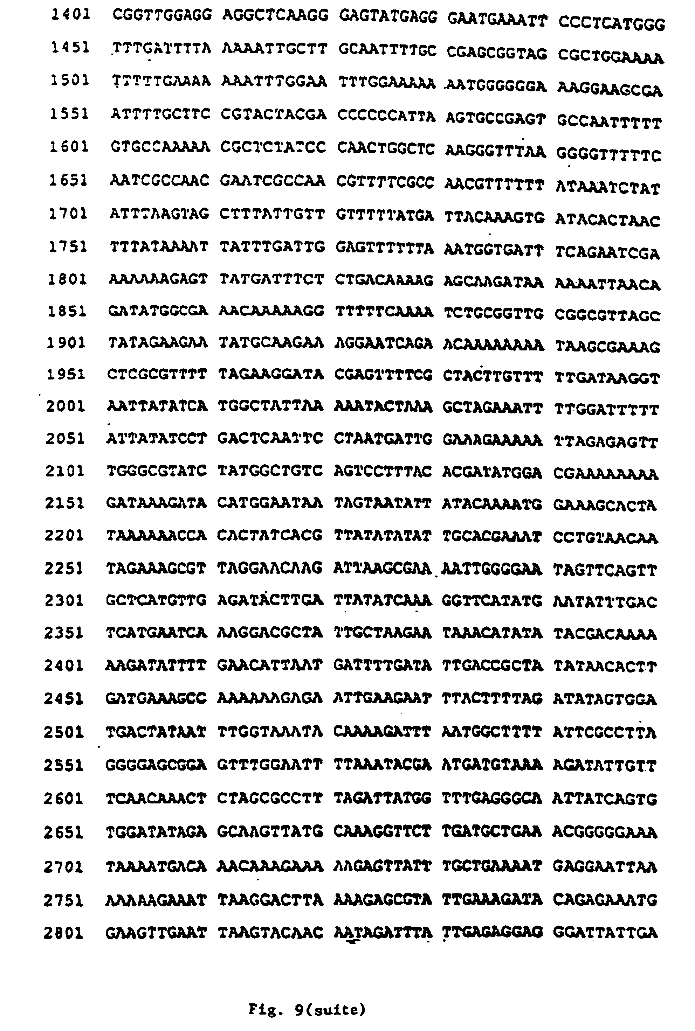

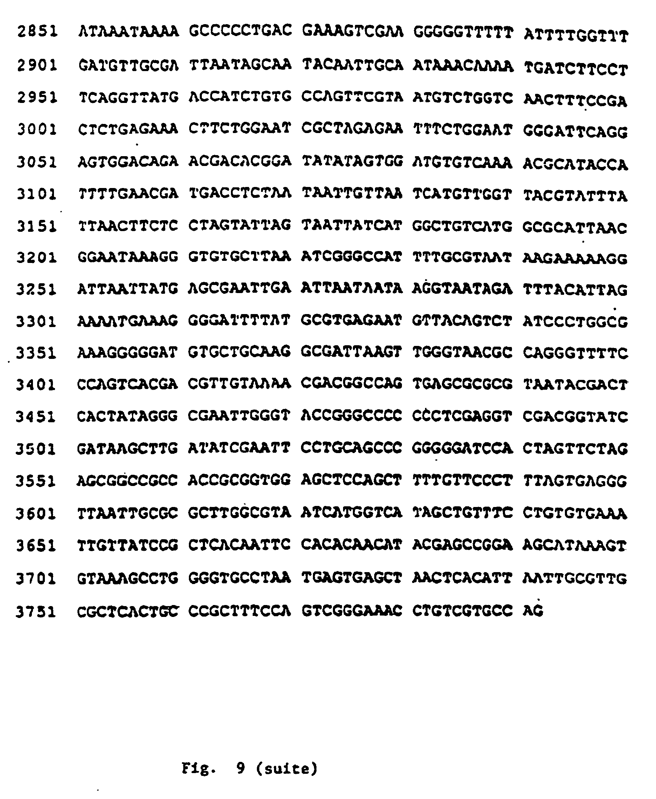

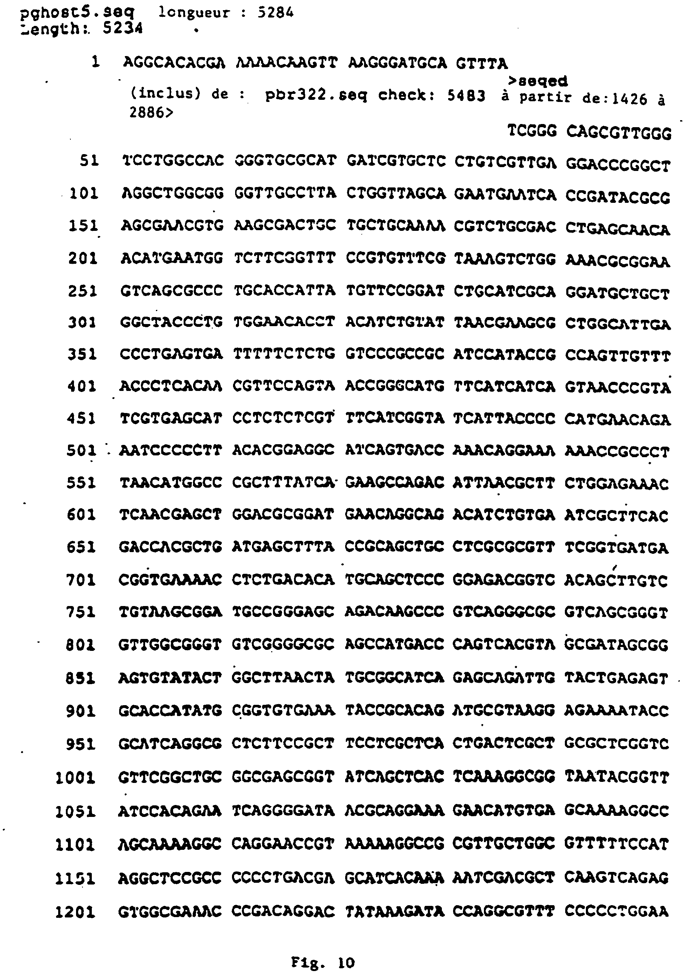

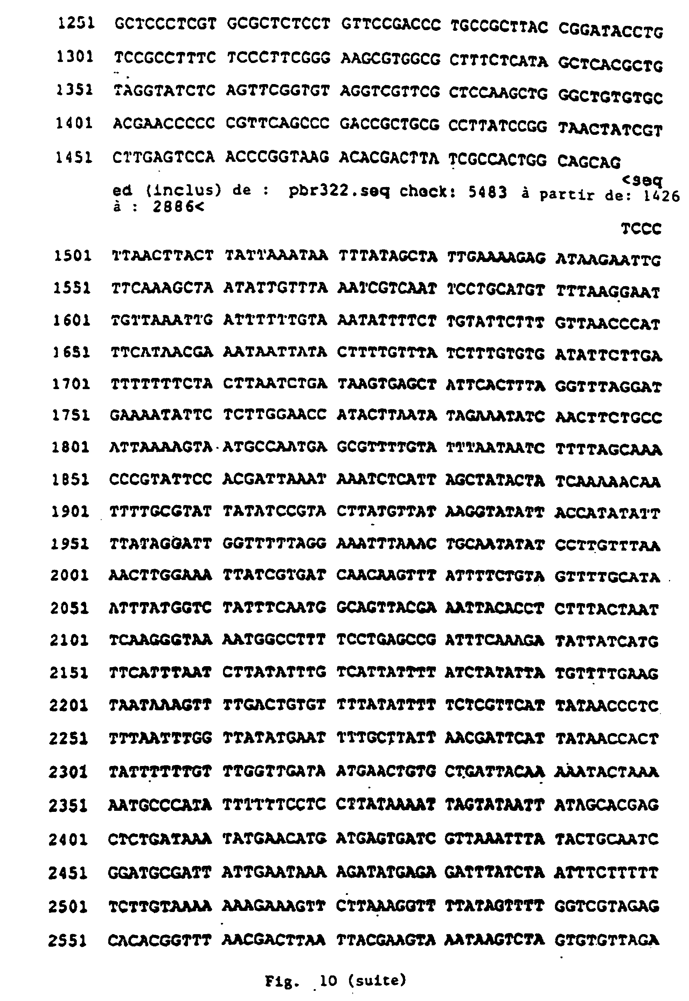

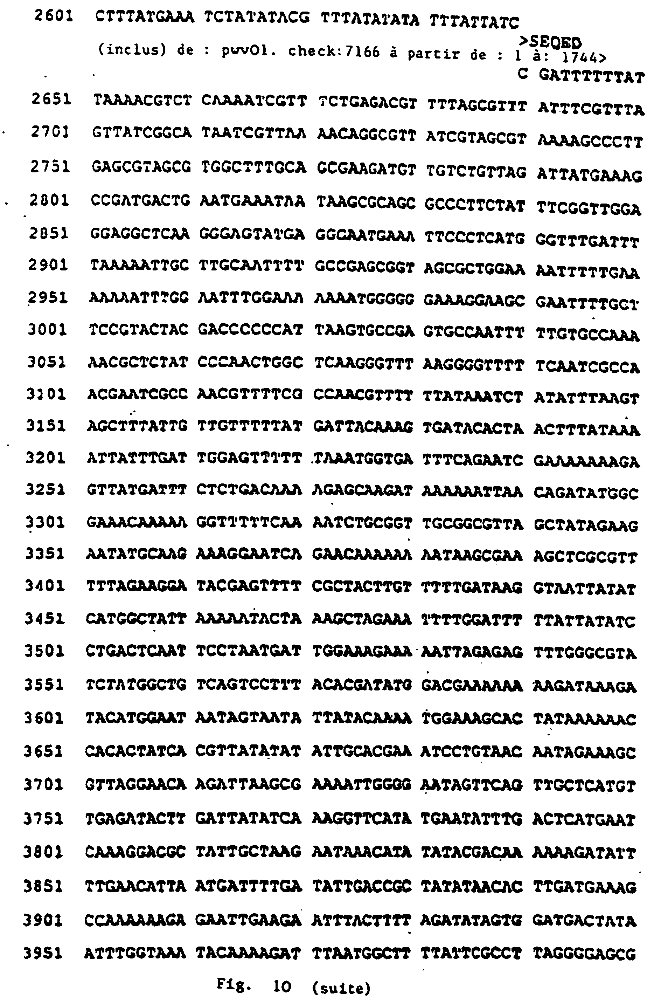

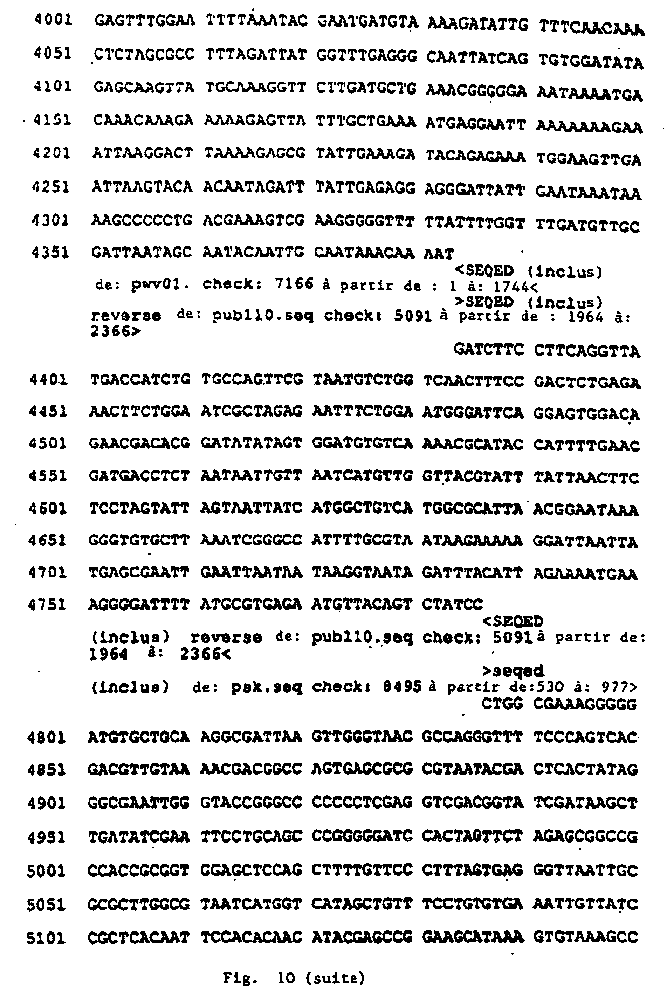

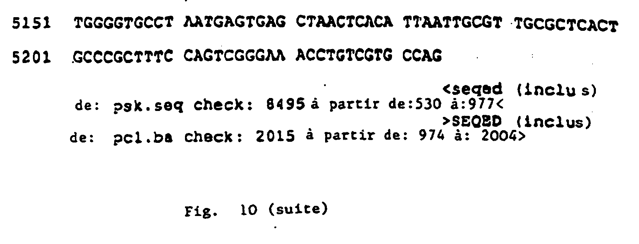

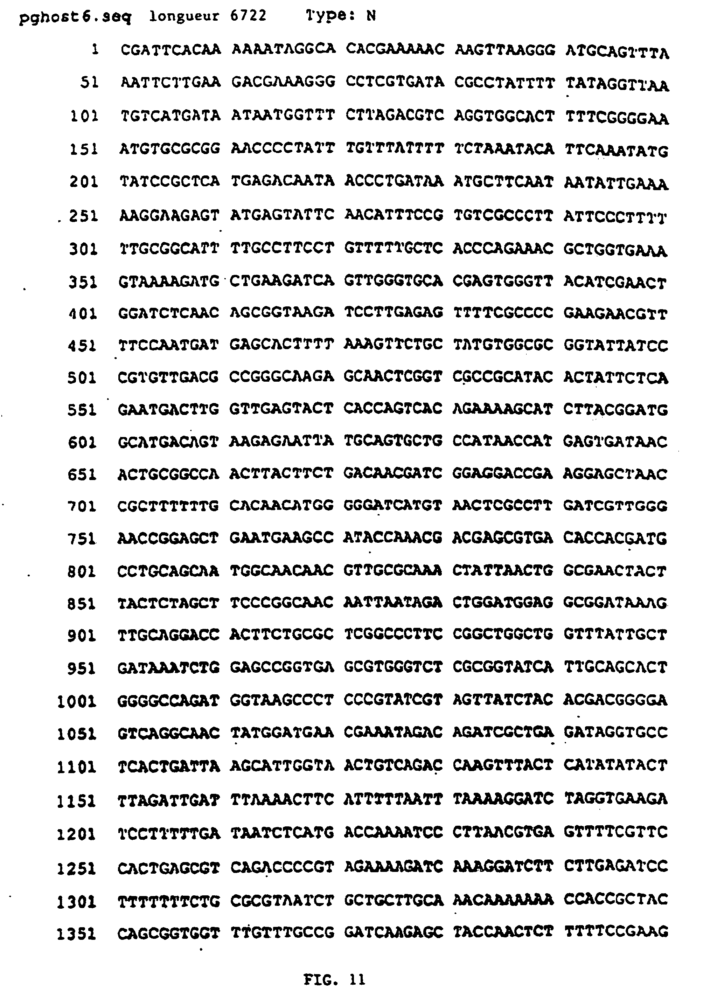

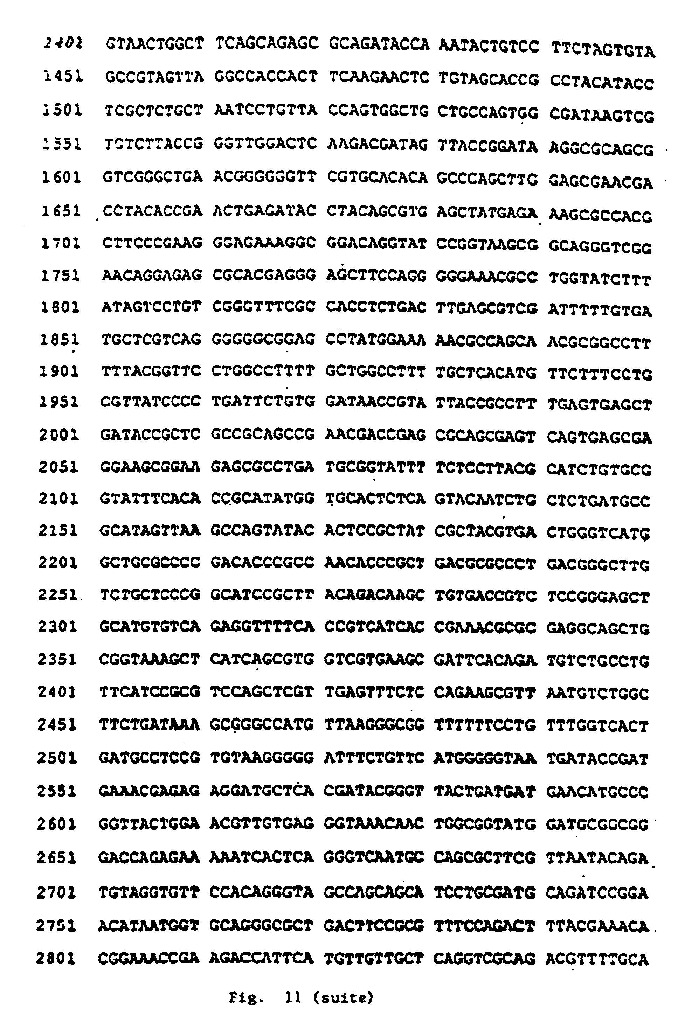

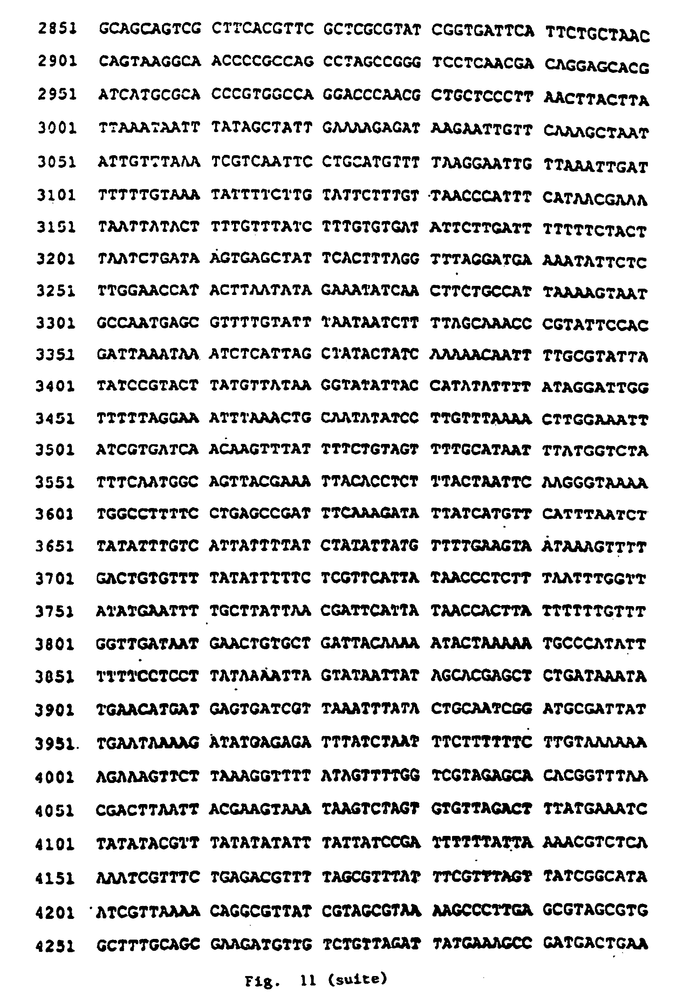

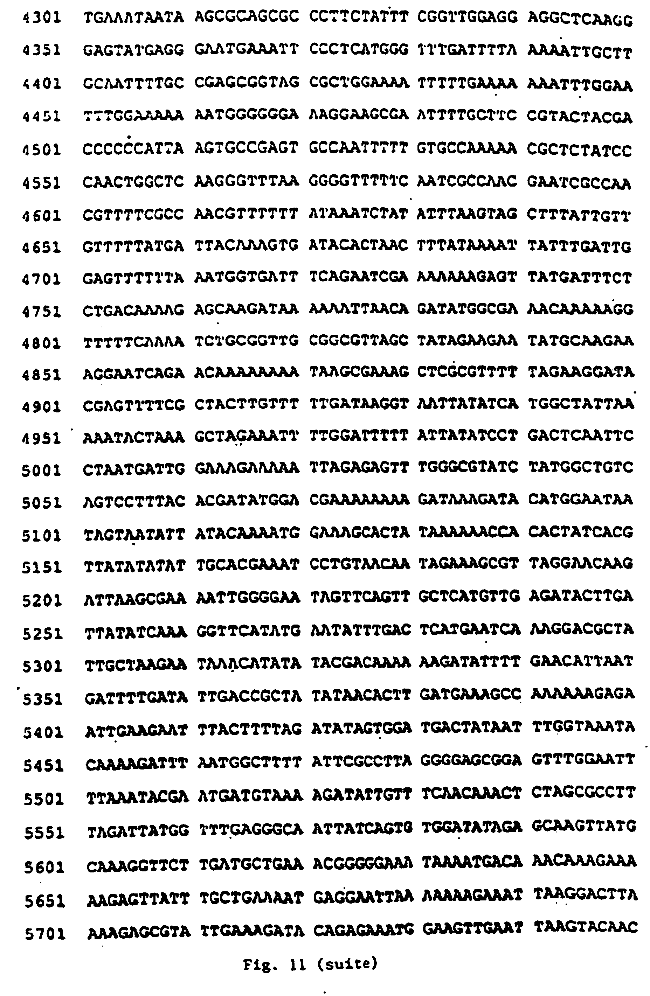

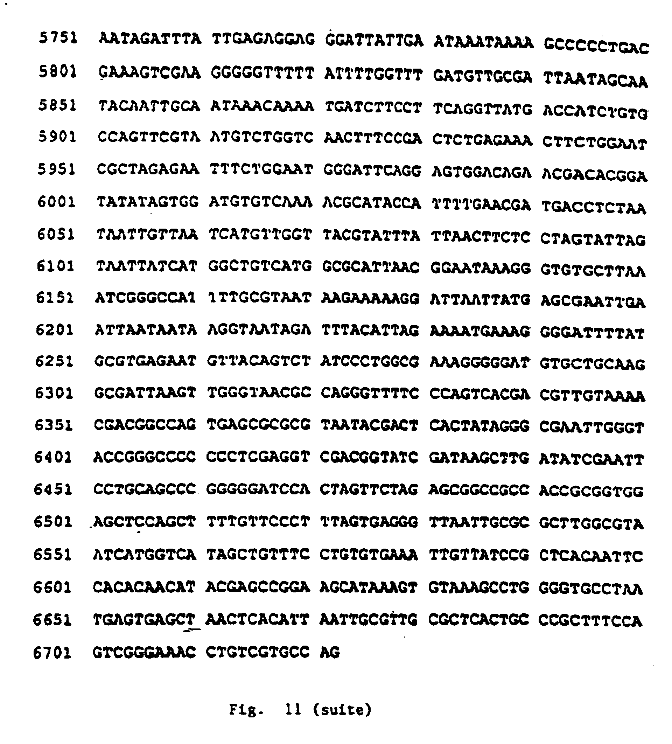

Plus particulièrement, des plasmides selon l'invention comportent une des séquences représentées sur une des figures 9, 10 ou 11, ou une séquence représentant au moins 80% d'homologie avec ces séquences.

Les outils génétiques ainsi développés permettent d'introduire et de stabiliser des gènes dans le chromosome bactérien.

On choisit par exemple d'appliquer un procédé par recombinaison homologue.

Pour cela, on utilise un réplicon thermosensible selon l'invention, qui comporte en outre au moins un fragment d'ADN homologue à l'ADN chromosomique de la bactérie que l'on veut modifier.

Selon l'un de ses aspects, la présente invention a pour objet un procédé d'inactivation d'un gène présent dans le chromosome d'une bactérie, caractérisé en ce que :
a) on introduit dans la bactérie, par transformation, le plasmide selon l'invention,
b) on cultive la bactérie sur milieu sélectif à une température inférieure à la température d'inhibition de l'origine de réplication,
c) on élève la température de culture à une température supérieure à ladite température d'inhibition,
d) on récupère les bactéries survivantes après plusieurs cycles de multiplication, à température d'inhibition de la réplication plasmidique.

L'étape d) permet de sélectionner les bactéries portant le marqueur plasmidique.

Le fragment chromosomique cloné dans le plasmide peut correspondre à un gène précis, qui est spécifiquement inactivé par intégration du plasmide au niveau de la copie chromosomique du gène. Dans la population bactérienne, seul ce site d'intégration sera trouvé.

Dans un autre mode de réalisation, l'ADN bactérien présent dans le plasmide pourra être choisi dans une banque de fragments chromosomiques pour le clonage, et il y aura intégration au hasard ; le site d'intégration du plasmide est différent d'une bactérie à l'autre et on réalise ainsi de la mutagénèse.

Le procédé peut également s'appliquer à un réplicon thermosensible porteur d'un transposon. On dispose de différents transposons pour mutagéniser le chromosome.

Le plasmide Ts est employé comme porteur d'un de ces transposons éventuellement modifié pour être actif chez L. lactis. Chaque transposon porte un gène marqueur (ex: gène de résistance). En appliquant le protocole précédemment décrit (a à c), on obtient, des cellules ayant intégré le transposon dans leurs chromosomes. On sélectionne ces cellules au moyen du marqueur du transposon. Dans le cas de la transposition, le plasmide n'est pas intégré dans le chromosome.

En variante, le plasmide vecteur selon l'invention comporte également un locus de mobilisation permettant la conjugaison. De préférence, ce locus de mobilisation est le locus ori T, extrait d'un plasmide de bactérie à gram positif, préférentiellement pouvant être extrait d'un plasmide de Streptococcus. Le plasmide vecteur portant ce locus peut être mobilisé et transféré par conjugaison chez des espèces bactériennes non transformables.

Le procédé d'inactivation d'un gène dans une bactérie fait alors intervenir les étapes suivantes :
a) on introduit dans la bactérie, par conjugaison, un plasmide selon l'invention, portant un locus de mobilisation et un fragment homologue au chromosome et/ou un transposon,
b) on cultive la bactérie sur milieu sélectif à une température inférieure à la température d'inhibition de l'origine de réplication,
c) on élève la température de culture à une température supérieure à ladite température d'inhibition,
d) on récupère les bactéries survivantes après plusieurs cycles de multiplication, à température d'inhibition de la réplication plasmidique.

Les bactéries obtenues à l'issue de l'étape d) ont subi un évènement de recombinaison ou de transposition et portent le marqueur du transposon ou du plasmide.

En variante, le plasmide vecteur selon l'invention, comporte également un réplicon actif chez E. coli. Le plasmide vecteur portant ce locus, et ces dérivés, peut être propagé chez E. Coli. Les constructions préparées et propagées chez E. coli à 37° C (grâce au deuxième réplicon) peuvent être ensuite transférées dans les bactéries lactiques dans lesquelles seul le réplicon Ts sera actif.

Dans les procédés décrits ci-dessus, après introduction du plasmide vecteur dans la bactérie par transformation ou conjugaison à l'étape a), on laisse le plasmide s'établir dans la population bactérienne, par réplication, à 28-30° C.

Le caractère de sélection est exprimé dans l'ensemble des bactéries. Quand la température s'élève au-dessus de 35° C, le plasmide sous forme libre devient incapable de se répliquer et se trouve donc perdu lors des divisions cellulaires. Seules les bactéries pour lesquelles ce plasmide s'est intégré par recombinaison dans le chromosome ou pour lesquelles le transposon s'est intégré dans le chromosome, conservent et transmettent l'information génétique portée par le plasmide ou le transposon et leur permettant de pousser sur milieu sélectif. On sélectionne ainsi les évènements d'intégration, de basse fréquence, en récupérant les bactéries se multipliant à 35-37° C sur milieu sélectif.

Lorsque le plasmide est intégré dans le chromosome, il présente une excellente stabilité, qui peut être de l'ordre de 99% après 75 générations à 37,5° C.

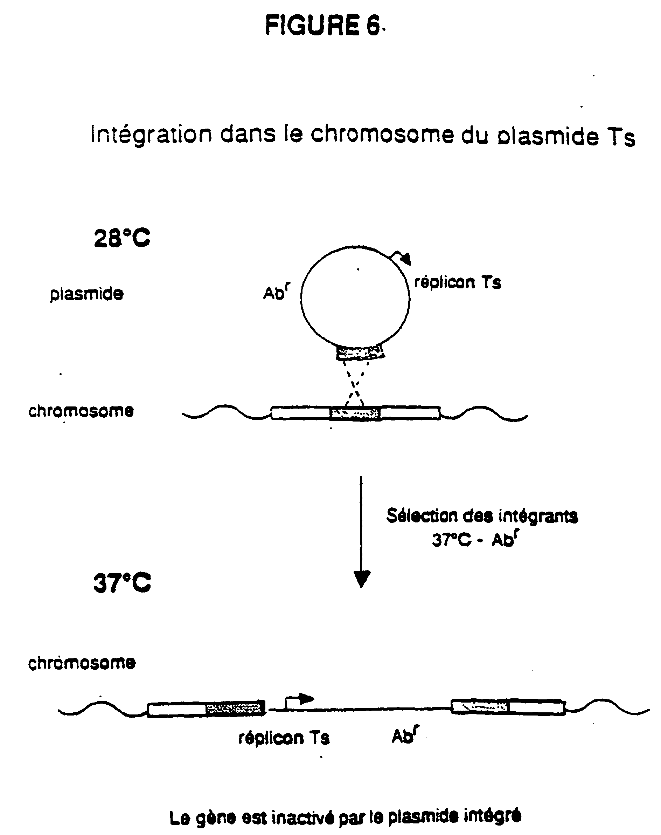

Le schéma suivi pour l'intégration du plasmide par recombinaison homologue est illustré à la figure 6.

La souche L. lactis VE 6002, contenant le plasmide pVE6002 selon l'invention a été déposée à la collection nationale de l'Institut Pasteur, 25-28 rue du Docteur Roux, Paris, sous le numéro 1-1179.

Selon un autre de ses aspects, l'invention a pour objet un procédé permettant l'introduction d'un gène hétérologue dans une bactérie. Pour sa mise en oeuvre, on utilise un plasmide vecteur thermosensible tel qu'il a pu être défini précédemment, et comportant en outre un gène codant pour une protéine d'intérêt, sous le contrôle des éléments nécessaires à son expression, et qui sont connus de l'homme du métier. Le cas échéant, ce gène pourra être porté par le transposon. On suit alors les étapes indiquées ci-après.
a) on introduit dans la bactérie, par transformation ou conjugaison un plasmide selon l'invention,
b) on cultive la bactérie sur milieu sélectif à une température inférieure à la température d'inhibition de l'origine de réplication,
c) on élève la température de culture à une température supérieure à ladite température d'inhibition,
d) on récupère les bactéries survivantes après plusieurs cycles de multiplication, à température d'inhibition de la réplication plasmidique.

L'étape d) permet de sélectionner les bactéries portant le marqueur du plasmide ou du transposon.

L'invention a également pour objet des bactéries contenant un plasmide selon l'invention, sous forme libre ou intégrée dans le chromosome.

De telles bactéries trouveront notamment des applications dans le domaine de l'industrie agroalimentaire, en particulier laitière, ou fromagère.

Dans certains des cas décrits précédemment, on souhaite pouvoir éliminer tout ou une partie du matériel génétique introduit dans le chromosome bactérien par le procédé selon l'invention.

Le procédé de recombinaison homologue permet deux étapes : la première consiste à sélectionner l'évènement d'intégration du plasmide, la seconde étape - qui est facultative - consiste à exciser du chromosome le réplicon et les marqueurs qui ne correspondent pas aux normes alimentaires.

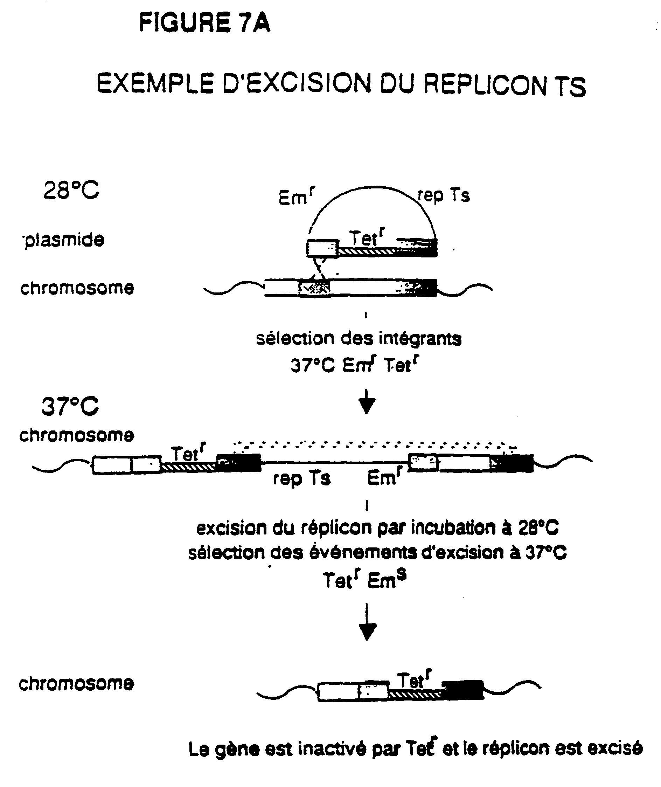

Excision du réplicon : l'intégration par recombinaison homologue crée des duplications de chaque côté du plasmide Ts (Fig. 7a). Il a été montré qu'un plasmide rolling-circle, réplicatif intégré dans le chromosome stimule fortement la recombinaison homologue entre les séquences avoisinantes. Lorsque le fragment chromosomique porté par le plasmide Ts contient un marqueur, les duplications créées par l'intégration permettent d'exciser le réplicon en laissant un gène chromosomique inactif (Fig. 7a). Expérimentalement, il s'agit de cultiver à 28° C la souche contenant le plasmide intégré (préalablement sélectionée à 37° C). A température permissive, la réplication reprend et stimule la recombinaison entre les séquences répétées aboutissant à la délétion du réplicon (Fig. 7a).

La présente invention a également pour objet un plasmide vecteur à réplication thermosensible présentant une ou plusieurs des caractéristiques déjà exposées, et dans lequel une région interne est dupliquée. Les deux séquences identiques sont placées de manière à encadrer la région que l'on souhaite éliminer. Un tel plasmide est alors utilisé dans un procédé d'inactivation d'un gène ou d'introduction d'un gène hétérologue par recombinaison dans le chromosome bactérien, tels que décrits ci-dessus.

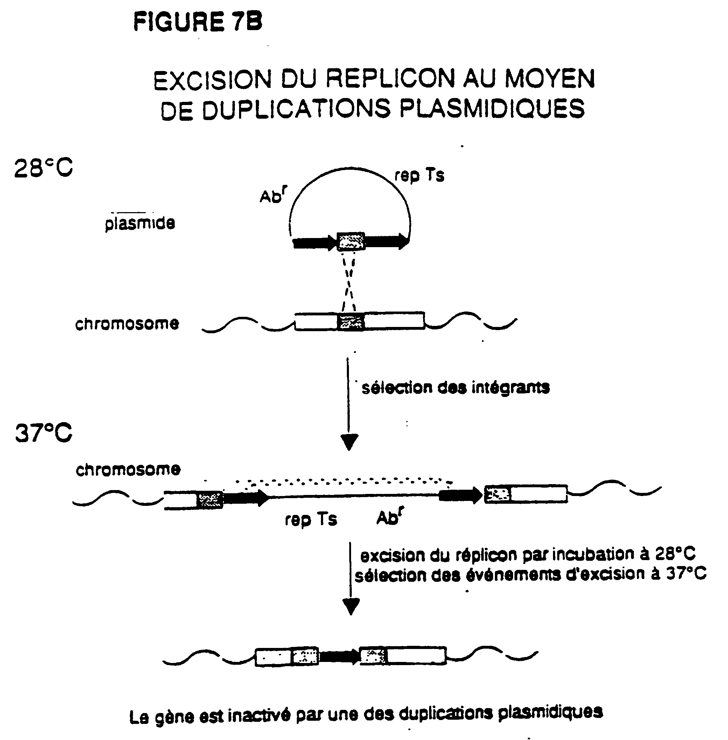

A l'issue de l'étape d), les bactéries survivantes sont à nouveau mises en culture à une température inférieure à la température d'inhibition, par exemple à 28-30° C, sur milieu non sélectif. En effet, un plasmide réplicatif stimule fortement la recombinaison homologue entre les séquences avoisinantes. La souche contenant le plasmide intégré préalablement sélectionnée à 35-37° C est cultivée à température permissive : la réplication plasmidique reprend, stimulant la recombinaison entre les séquences répétées. Le réplicon et les marqueurs incompatibles avec, par exemple, une utilisation agroalimentaire de ce système, sont excisés, avec rétention éventuelle du gène modifié. La sélection des bactéries ayant excisé les marqueurs indésirables se fait après étalement à température non-permissive. Ce mécanisme est illustré sur la figure 7b.

Les exemples qui suivent sont destinés à illustrer l'invention sans aucunement en limiter la portée.

On se réfèrera aux figures suivantes :

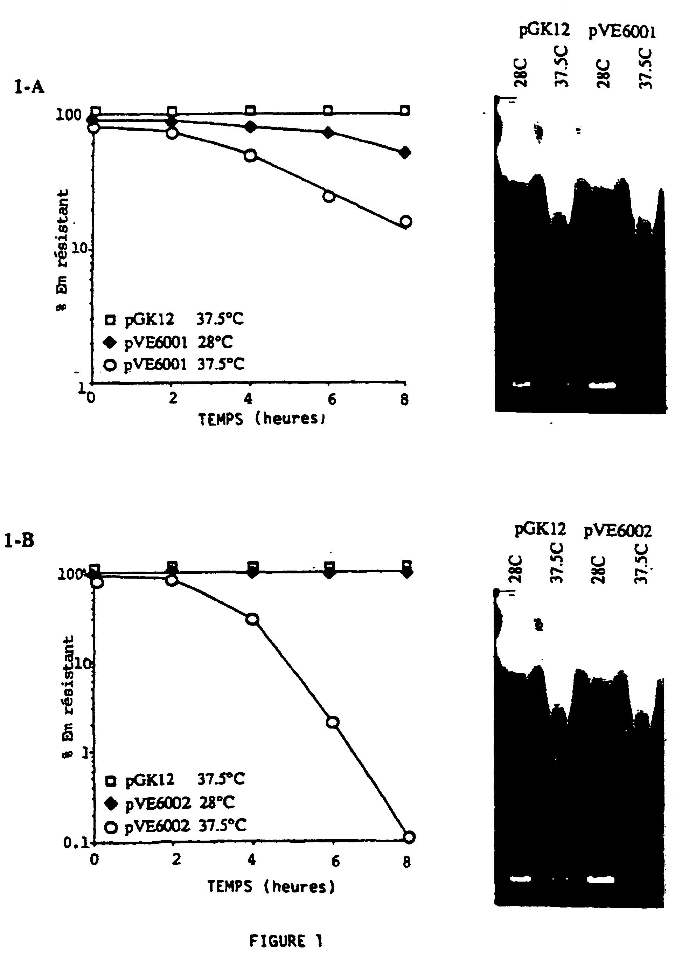

- FIGURE 1 :: cinétique de perte et analyse du nombre de copies de pVE6002 selon l'invention et de pVE6001 non Ts. L. lactis subsp. lactis IL 1403 portant le plasmide pGK12, pVE6001 ou pVE6002 sont cultivés à 28° C ou 37,5° C. Après différents temps de culture, des échantillons sont prélevés pour être cultivés à 28° C sur des milieux sélectifs et non-sélectifs. 100 colonies sont repiquées à partir du milieu non-sélectif sur le milieu sélectif (Em 5 µg/ml) pour évaluer la proportion de cellules contenant un plasmide dans la population. Les extractions d'ADN total sont faites sur les cultures à 28° C ou 37,5° C, sans sélection, pendant 5h 30.
- FIGURE 2 :: Plasmide hybride de pGK12 et pVE6002. pVE6043 est constitué du fragment Sacl-Thal, de 994 pb, de pGK12, lié au fragment Thal-Sacl de 3384 pb de pVE6002. pVE6044 contient la paire réciproque, le fragment Sacl-Thal de 994 pb de pVE6002, lié au fragment Thal-Sacl de 3384 pb de pGK12. Les traits fins correspondent à l'ADN de pGK12 ; les traits épais pointillés, à l'ADN de pVE6002.
- FIGURE 3 :: Localisation de la mutation Ts dans le gène RepA de pVE6002. Le fragment Thal-Rsal de pVE6002, contenant le gène RepA a été séquencé sur les deux brins. La séquence montre quatre mutations aux positions 972, 977, 980, 987 alors que le reste de la séquence ne diffère pas de la séquence publiée pour le réplicon parental pWV01.
- FIGURE 4 :: Description des dérivés thermosensibles. pVE6006 est construit par insertion du fragment PvuII de 445 pb de pBluescript SK+ dans le site ClaI de pVE6002. pVE6007 provient d'une délétion Sacl de pVE6006, conduisant à la perte du gène de résistance à l'érythromycine. pVE6004 est construit par insertion du fragment PvuII de 445 pb de pBluescript SK+ dans le fragment ClaI-HpaII de pVE6002 dépourvu de gène de résistance au chloramphénicol.

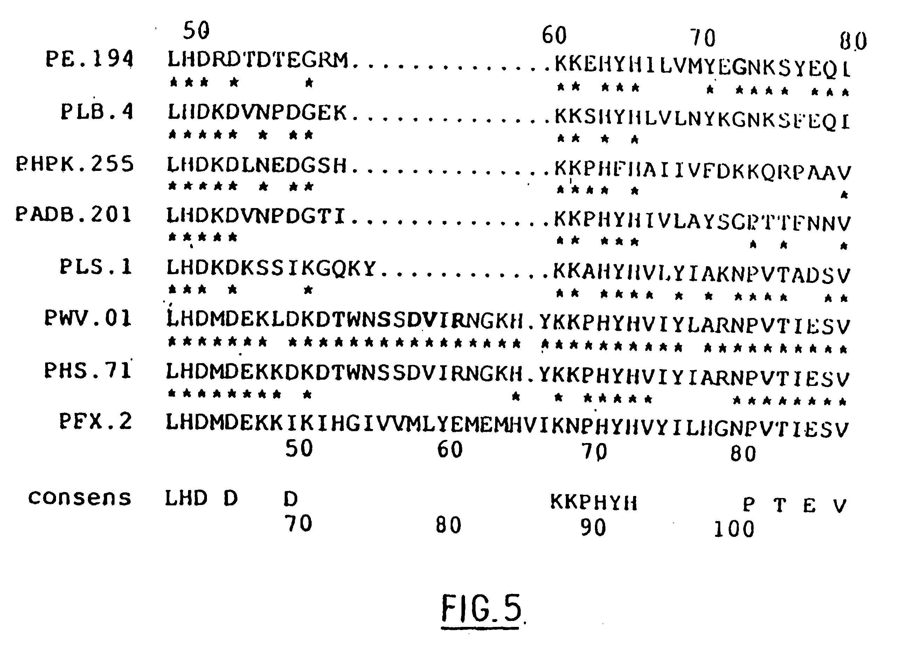

- FIGURE 5 :: Comparaison des protéines analogues à Rep de PE.194.
- FIGURE 6 :: Schéma du procédé d'inactivation d'un gène.
- FIGURE 7a :: Schéma d'un exemple d'excision du réplicon Ts en deux étapes.
- 7b :: Schéma de l'excision du réplicon au moyen de duplications plasmidiques.
- FIGURE 8 :: Construction des plasmides pG⁺host5 et pG⁺host6 à partir du plasmide pG⁺host4 (ou pVE6004) : le plasmide pG⁺host5 est construit par insertion du fragment Ava I - Alw N 1 de pBR 322 (qui contient l'origine de réplication de pBR 322) dans le pG⁺host4 linéarisé par Nsi 1.
pG⁺host6 est construit par insertion du fragment Ava 1 - Eco R 1 de pBR 322 (qui contient l'origine de réplication de pBR 322 et le gène de résistance à l'ampicilline) dans le pG⁺host4 linéarisé par Nsi I.
- FIGURE 9 :: Séquence nucléotidique de pG⁺host4.
- FIGURE 10 :: Séquence nucléotidique de pG⁺host5.
- FIGURE 11 :: Séquence nucléotidique de pG⁺host6.

### Exemple 1 : Préparation et caractérisation d'un plasmide vecteur thermosensible

### MATERIELS ET METHODES

Les travaux ont été réalisés au Laboratoire de Génétique Microbienne, Institut de Biotechnologie, INRA, 78352 Jouy-en-Josas cedex France.

### Souches bactériennes, plasmides et conditions de culture.

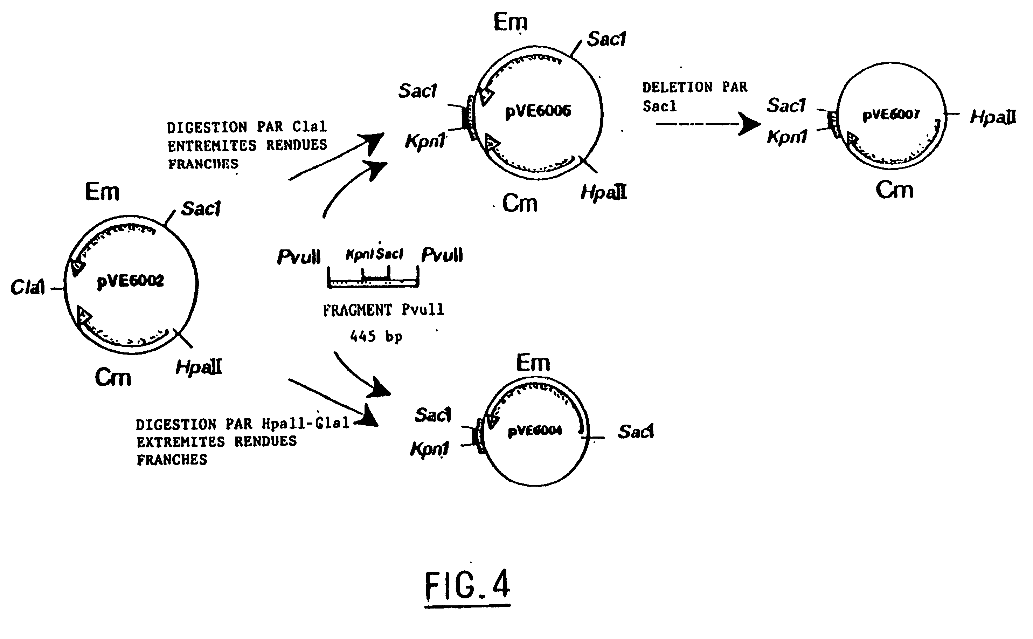

Les plasmides et les souches bactériennes utilisées sont indiqués dans le tableau 1. Les constructions de pVE6043 et pVE6044 sont décrites dans la figure 2 ; les plasmides pVE6004, pEV6006 et pVE6007 sont présentés à la figure 4. pVE6004 (ou pG⁺host4) est construit par insertion d'un fragment d'ADN PvuII de 445 pb dans le fragment ClaI-HpaII de 3340 pb à extrémité franche de l'isolat Ts original, dépourvu du gène de résistance Cm. Le fragment PvuII de 445 pb contient un site de multiclonage, les promoteurs T7 et T3, et les sites pour M13 - 20, T7, T3 et des amorces inverses qui permettent le séquençage direct à partir du vecteur. Ce plasmide est thermosensible dans tous les hôtes testés, y compris chez E. coli et doit être maintenu à 28° C.

E. coli et Bacillus subtrlis ont été cultivés en milieu LB. L. lactis subsp. lactis (L. lactis) est cultivé sur milieu M17, dans lequel on a remplacé le lactose par du glucose. On a utilisé respectivement du chloramphénicol (Cm) à 5 µg/ml pour L. lactis et B. subtilis et de l'érythromycine (Em) à 5 µg/ml et 0,5 µg/ml respectivement. Le Cm, l'azaerythromycine et l'érythromycine ont été utilisés à des concentrations finales respectives de 15 µg/ml, 100 µg/ml et 150 µg/ml pour E. coli. Clonage moléculaire, compétence et procédure de transformation.

Des enzymes du commerce ont été utilisées comme indiqué par les fournisseurs. Des mini-lysats de cellules entières et du DNA plasmidique ont été préparés ainsi qu'il est décrit dans la littérature. L'induction de compétence et la transformation d'E. coli et de B. subtilis ont été effectuées par des procédures standards (Hanahan, 1985, ou Niaudet et al, 1979). Les souches de L. lactis ont été électrotransformées comme décrit par Langella et Chopin, 1989a et modifié selon la procédure de Holo et Nes. 1989.

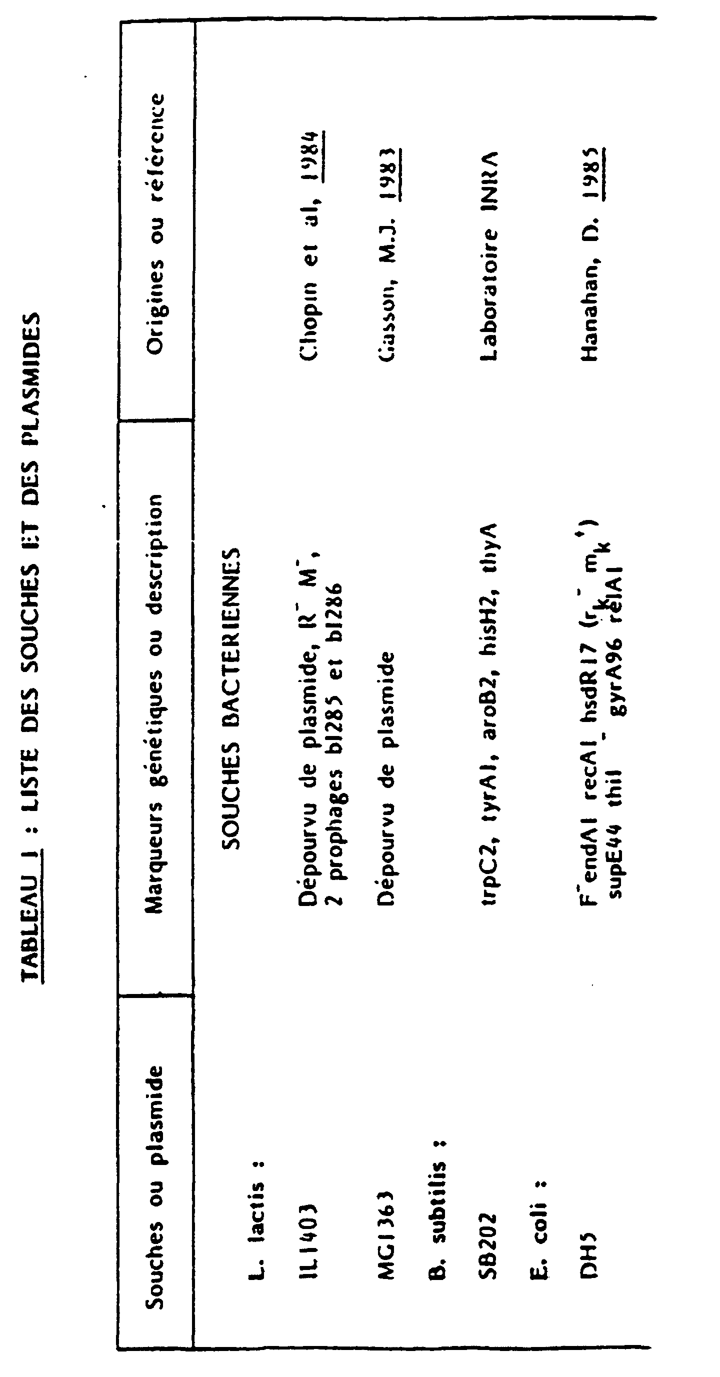

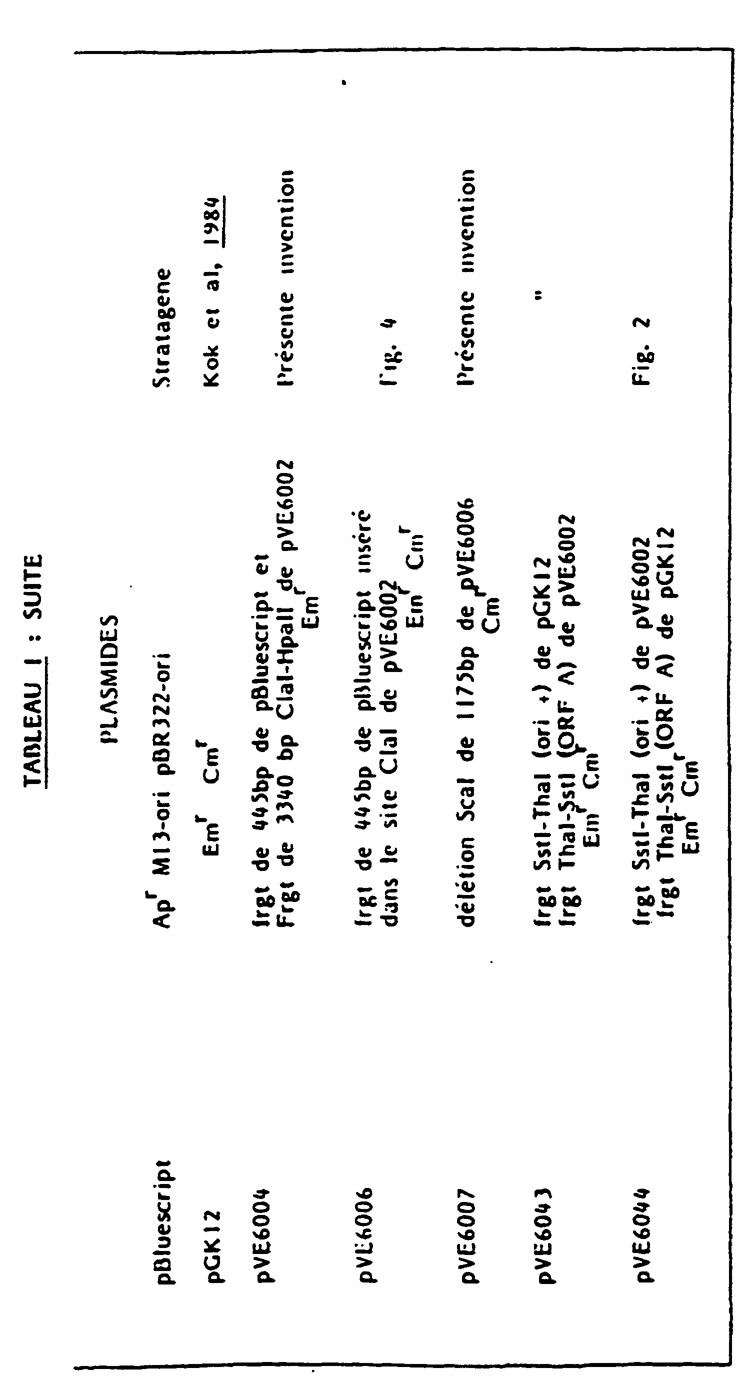

### Mutagénèse des plasmides.

La mutagénèse par hydroxylamine a été effectuée sur l'ADN du plasmide pGK12 dans les conditions décrites par Thomas, 1987. Après 110 et 120 minutes de traitement à 70° C, l'hydroxylamine est éliminé par précipitation du DNA à l'isopropanol.

### Séquençage du DNA.

Pour le séquençage du DNA, on a cloné le fragment ThaI (756pb)-Rsal (1620 pb) de pVE6002, dans le plasmide pBluescript. On génère une série de clones se recouvrant, par utilisation d'exonucléase III et de nucléase de haricots mung (Stratagène). Le fragment ThaI (756pb)-Ndel (1140pb) de la préparation du plasmide pGK12 utilisé pour la mutagénèse est également séquencé par la même procédure.

On réalise le séquençage du DNA par la méthode de terminaison de chaînes didéoxy sur des ADN doubles brins avec le Kit de séquençage Taq Dye Primer Cycle (Applied Biosystem) en utilisant un appareil PCR Perkin Elmer. Les réactions de séquençage sont initiées avec des oligonucléotides fluorescents (Applied Biosystem) et sont analysées sur un séquenceur automatique (séquenceur 370 A DNA, Applied Biosystem). Les séquences obtenues ont été déterminées sur les deux brins.

### RESULTATS

### Isolement du mutant.

Le plasmide utilisé dans ces expériences, pGK12, est un dérivé de pWV01 contenant deux marqueurs de résistance aux antibiotiques (KoK et al, 1984). 10 µg d'ADN plasmidique sont mutagénisés in vitro par de l'hydroxylamine et introduits par électroporation dans la souche de lactococcus IL1403, après élimination de l'agent mutagène. L'efficacité de la mutagenèse est évaluée par la diminution de viabilité du plasmide et par l'apparition de mutants sensibles à l'érythromycine ou au chloramphénicol. Après 110 à 120 minutes de traitement, la viabilité du plasmide chute à moins de 0,1 % et environ 10 % des transformants contiennent des plasmides sensibles à l'un des antibiotiques. Ces conditions de mutagénèse sont choisies pour rechercher des plasmides thermosensibles, identifiés par réplique des transformants obtenus à 28° C sur un milieu contenant de l'érythromycine incubés à 37,5° C. Deux candidats thermosensibles, dénommés pVE6001 et pVE6002, sont obtenus par criblage d'environ 5000 clones. Leurs nombres de copies plasmidiques sont comparés, et la perte à 37.5° C est déterminée.

### Caractérisation du mutant : pVE6001 est un mutant non Ts.

Le plasmide pVE6001 est plus instable que pGK 12 à 28° C, et cette déficience devient plus prononcée à 37,5° C. Cependant, 7 % des bactéries contiennent encore le plasmide après 8 heures de croissance non sélective à 37,5° C, ce qui suggère qu'une réplication a lieu dans les conditions restrictives (figure 1A, gauche).

Par rapport à pGK12, le nombre de copies de pVE6001 apparaît diminué à 28° C et 37,5° C, avec ou sans sélection (figure 1A), ce qui pourrait expliquer sa stabilité inférieure. Il est possible que la perte du plasmide à des températures élevées, soit due à des changements physiologiques chez l'hôte à des températures supérieures, et non à la thermosensibilité du plasmide.

### Caractérisation du mutant : pVE6002 est un mutant thermosensible au dessus de 35° C.

Les mesures de la stabilité du plasmide pendant la croissance sans antibiotique, révèlent que pVE6002 est aussi stable que pGK 12 à 28° C, mais est perdu de façon drastique à 37,5° C (figure 1B). La perte rapide de pVE6002 à 37,5° C suggère que la réplication est bloquée immédiatement après le changement de température. Après 8 heures de croissance. il ne reste qu'environ 0,1 % de cellules résistantes à l'érythromycine. Les nombres de copies de pGK12 et de pVE6002 sont similaires à 28° C, avec et sans sélection ; cependant, après 5 heures à 37,5° C, pVE6002 est indétectable alors que le nombre de copies de pGK12 est à peu près le même (figure 1B). On peut conclure de ces expériences que la mutation sur pVE6002 constitue réellement une déficience thermosensible de réplication.

La température minimum permettant la perte de pVE6002 a été déterminée. La souche IL1403 contenant pVE6002 a été testée pour la perte plasmidique pendant 8 heures de croissance non sélective à 28° C, 30° C. 33° C, 35° C et 37,5° C (tableau 2).

**TABLEAU 2 :**

| **Pourcentage de cellules Em**^{**r**} **dans la population** | | | | | |
|---|---|---|---|---|---|
| Temps (Heures) | Température de croissance | | | | |
| | 28° C | 30° C | 33° C | 35° C | 37,5° C |
| 0 | 100 | 100 | 100 | 100 | 100 |
| 2 | 100 | 100 | 99 | 97 | 98 |
| 4 | 100 | 100 | 48 | 47 | 38 |
| 6 | 100 | 100 | 9 | 3 | 4 |
| 8 | 100 | 99 | 5 | 1 | 1 |

Une culture d'une nuit en M17 avec Em, de IL1403 portant pVE6002 est diluée dans du milieu sélectif neuf et mise à pousser 3 heures à 28° C. La culture est alors diluée 10.000 fois dans un milieu non-sélectif et incubée à différentes températures. A des intervalles de temps variés. des échantillons sont prélevés et placés sur M17 à 28° C. Pour chaque point de température et de temps, la perte du plasmide est évaluée en repiquant une centaine de colonies sur des boîtes de milieu sélectif (Em) à 28° C.

On a trouvé que la perte plasmidique est équivalente à 37,5° C et 35° C. Une perte partielle du plasmide est déjà observée à 33° C, alors que le plasmide était stable à 28° C et 30° C. Ainsi, les cellules contenant pVE6002 peuvent perdre ce plasmide par élévation de la température à 35° C ou plus.

On a aussi introduit pVE6002 dans une autre souche de Lactococcus, MG 1363 (Gasson, 1983), qui est distincte de IL1403 par comparaison des profils d'électrophorèse en champ pulsé. L'analyse des séquences indique que MG1363 est probablement une souche L. lactis subsp. cremoris (Godon et al, 1992). pVE6002 montre la même thermosensibilité dans cet environnement, démontrant que le phénotype du mutant plasmidique n'est pas lié à la souche.

### Large spécificité d'hôte et phénotype Ts de pVE6002.

Un plasmide Ts peut être un véhicule de clonage utile dans d'autres organismes. Ainsi le comportement thermosensible de pVE6002 a été examiné chez B. subtilis et E. coli. Ces souches ont été choisies comme représentatives du large spectre d'hôte du réplicon original pWV01. L'ADN plasmidique a été introduit dans les deux espèces par transformation et sélection à 28° C. Etant donné que B. subtilis et E. coli ont des températures de croissance maximales supérieures à celles de L. lactis subsp., la réplication de pVE6002 a été testée à 28° C, 37° C et 42° C. Les résultats montrent que pVE6002 est thermosensible chez les deux hôtes. Il est probable que pVE6002 conserve ses propriétés de thermosensibilité chez le large spectre d'hôtes chez lequel il peut être établi.

### Cartographie de la mutation Ts.

La séquence d'ADN de pWV01 montre la présence d'une origine-plus et de quatre ORF. Leenhouts déduit de sa similarité avec des ADN plasmidiques mieux caractérisés, que l'ORF A code pour la protéine de réplication (RepA) responsable de la coupure d'un brin d'ADN à l'origine-plus. Des homologies supplémentaires suggèrent que l'ORF C pourrait réguler l'expression de RepA. Des fonctions n'ont pas encore été clairement attribuées à ORF B et ORF D, bien que l'on sache que cette dernière n'est pas nécessaire à la réplication.

Afin de localiser la mutation qui confère la thermosensibilité à pVE6002, on a construit des plasmides hybrides associant des parties du réplicon thermosensible et des parties non mutées. pVE6043 est constitué d'un fragment de pGK12 contenant l'origine-plus, ORF B et ORF C, et d'un fragment pVE6002 (Ts) contenant l'ORF A (RepA) dépourvu de son promoteur, l'ORF D et les marqueurs de résistance à Em et Cm (on utilise les sites de restriction Sacl et Thal, figure 2). Cet hybride est perdu à 37,5° C, au même taux que pVE6002, alors que l'hybride réciproque (pVE6044) est maintenu avec la même stabilité que pGK12 (figure 2). Ainsi, la mutation conférant la thermosensibilité à pVE6002 se trouve dans le fragment d'ADN codant pour RepA, ORF D et les marqueurs aux antibiotiques. Etant donné que l'ORF D n'est pas indispensable et que les marqueurs aux antibiotiques ne sont pas candidats, on peut conclure que la fonction thermosensible est la protéine RepA.

### Données de séquençage.

Afin de localiser la mutation Ts, on a séquencé le fragment de 864 pb codant pour la protéine RepA de pVE6002. On a identifié quatre mutations, aux positions 972, 977, 980 et 987 (figure 3). On a confirmé que la région correspondante du plasmide parental pGK12 qui a été utilisé pour la mutagénèse est identique à la séquence publiée pour pWV01 (Leenhouts et al, 1991). Les quatre mutations sont des transitions de G à A, correspondant à l'effet mutagène connu de l'hydroxylamine. Chaque changement de base a pour résultat une altération d'un acide aminé (figure 3) dont l'une, Val en Ile est conservative. La contribution d'une ou plusieurs de ces altérations peut être impliquée dans le phénotype Ts.

### Dérivés du plasmide Ts.

Dans un but de clonage, des dérivés du plasmide Ts initial pVE6002 ont été développés (figure 4). Ces dérivés sont modifiés pour contenir soit les deux résistances aux antibiotiques (Em et Cm) soit seulement l'une (Em ou Cm) et tous ont une séquence multisite dérivée du plasmide pBluescript SK+.

### Excision du réplicon

Des évènements de double échange réciproque ont pu être obtenus à partir d'un plasmide dérivé de pVE6002 portant une région d'homologie avec le chromosome bactérien interrompue par un gène de résistance à un antibiotique (Ab^{r}) comme illustré sur la figure 7a. Après introduction du plasmide dans la bactérie à 28° C, une première étape consiste à sélectionner les intégrants dans le chromosome par culture à 37° C sur milieu contenant l'antibiotique. La région d'homologie est dupliquée à la suite de l'intégration (Figure 7a). Lors d'une seconde étape, on obtient l'excision du réplicon par incubation à 28° C pour permettre à la réplication plasmidique de reprendre et stimuler un second évènement de recombinaison. Les évènements d'excision sont sélectionnés par culture à 37° C ; le gène chromosomique est inactivé par le gène Ab^{r}.

### Exemple 2 : Intégration du plasmide thermosensible dans le chromosome de L. lactis

Les souches bactériennes et les plasmides utilisés dans cette étude sont présentés au tableau 3. Les souches d'Escherichia coli sont cultivées dans du bouillon LB. L. lactis est cultivé et étalé sur un bouillon M17⁺ glucose ou sur un milieu minimum quand il est testé pour le phénotype ilv. L'Erythromycine (Em) est ajouté à une concentration de 5 microgrammes/ml pour L. lactis subsp. lactis et 150 microgrammes/ml pour E. coli, la tétracycline est utilisée à une concentration de 12,5 microgrammes/ml pour L. lactis. L'électroporation de L. lactis subsp. lactis (Appl. Env. Microbiol. 55, 3119-3123, 1989) donne entre 10⁵ et 10⁶ transformants par microgramme d'ADN plasmidique pour IL1403 et environ 10² transformants par microgramme d'ADN plasmidique avec NCDO2118, la souche ilv⁺ utilisée pour les expériences de remplacement du gène. E. coli est transformée par la méthode décrite par Hanahan (1985, DNA cloning: A practical approach Vol 1: 109-135, IRL Press Ed Glover).

**Tableau 3**

| SOUCHE | MARQUEURS GÉNÉTIQUES OU DESCRIPTION | SOURCE |
|---|---|---|
| | | |
| L lactis : NCD02118 | isolat naturel | * |
| | | |
| E. coli : TG1 | supE hsdΔ5 thi Δ(lac-proAB) F [traD36proAB⁺ lacI^{q} lacZΔM15] | Sambrook et al |
| | | |

| PLASMIDES | | |
|---|---|---|
| pG+host4 ou pV6004 | dérivé thermosensible de pGK12, Em^{r} | ** |
| | | |
| pG⁺host5 | frag. Nsil de pG⁺host4 lié au fragment 1,46 Kb AvaI-AlwNI de pBR322 Em^{r} | ** |
| | | |
| pVE7021 à pVE7034 | produit de restriction SmaI-HindIII de pG⁺host5 lié à un fragment EcoRV-HindIII aléatoire du chromosome de IL1403 | ** |
| | | |
| pIL515 | frag. EcoRI, ilv de 3,9 Kbde IL1403 dans pBluescript, Ampr | ** |
| | | |
| pVE7009 | frag. EcoRI de de 3,9 Kb de pII515 lié à pG⁺host5 coupé par EcoRI | ** |
| pVE7009R | même construction que pVE7009, inséré dans l'orientation opposée | ** |
| | | |
| pVE7015 | délétion SphI-EcoRV de pVE7009R laissant un frag. ilv de 3362 pb | ** |
| | | |
| pVE7014 | délétion StyI-EcoRV de pVE7009R laissant un frag. ilv de 2904 pb | ** |
| | | |
| pVE7010 | délétion Clal de pVE7009R laissant un frag. ilv de 2552 bp | ** |
| | | |
| pVE7016 | délétion XcmI-EcoRV de pVE7009R laissant un frag. ilv de 1912 bp | ** |
| | | |
| pVE7013 | délétion AatII-EcoRV de pVE7009R laissant un frag. ilv de 1206 bp | ** |
| | | |
| pVE7011 | délétion HindIII de pVE7009R laissant un frag. ilv de 497 bp | ** |
| | | |
| pVE7012 | délétion PstI de pVE7009R laissant un frag. ilv de 356 bp | ** |
| | | |
| pVE7017 | délétion PflMI-EcoRV de pVE7009R laissant un frag. ilv de 330 bp | ** |
| | | |
| pIL500 | frag. 18,5 Kb Xbal ilv de NDC02118 chromosome dans pIL253 | Godon et al |
| pIL1202 | frag. XbaI de pG⁺host4 contenant les extrémités XbaI-BgIII de 1,1 Kb et EcoRI-XbaI de 2,5 Kb du frag. de 18,5 Kb de pIL500 lié au frag. BamHI de 4 Kb du gène Tet M | |
| | | |
| pIL1261 | frag. 2,3 Kb XbaI-EcoRI de pIL500 interrompu par un gène Tet M, BamHI de 4 Kb inséré au site BgIII et lié à XbaI-EcoRI pBluescript | |
| | | |
| pIL1263 | XbaI-EcoRI pG+host4 lié au frag. XbaI-EcoRI de pIL1261 de 6,3 Kb | |

| | | |
|---|---|---|
| * : National Collection of Dairy Organisms | | |
| ** : Présente invention | | |

### Construction des plasmides pour l'intégration

### a) construction du vecteur

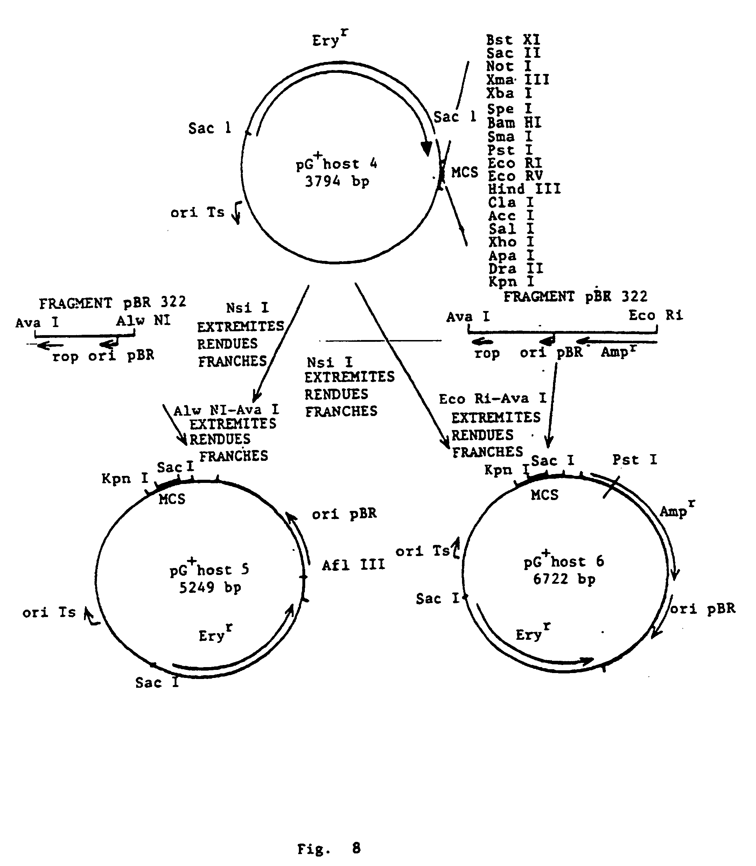

Le plasmide pG⁺host4 (ou pVE6004) est un dérivé Ts de pWVO1 préparé selon l'exemple 1. Pour faciliter le clonage chez E. coli, le fragment de 1,4 Kb contenant l'origine de pBR322 est inséré dans pG⁺host4. Le plasmide pG⁺host5 est construit par insertion du fragment Ava 1 - Alw N 1 de pBR 322 (qui contient l'origine de réplication de pBR 322) dans le pG⁺host4 linéarisé coupé parNsi 1. Sa structure est représentée sur la figure 8. Le plasmide obtenu, appelé pG⁺host5 (Appligène, Illkirch, France) est utilisé pour tous les clonages. L'activité de l'origine de pBR322 permet son maintien à 37° C chez E. coli et l'origine Ts maintient pG⁺host5 à 28° C dans les bactéries gram positif.

### b) clonage de fragments chromosomiques aléatoires dans pG⁺host 5.

L'ADN chromosomique de la souche IL1403 est digéré par EcoRV et HindIII. Des fragments chromosomiques d'une taille comprise entre 0,9 Kb et 1,4 Kb, sont purifiés à partir de gels d'agarose et liés avec pG⁺host5 traité par SmaI-HindIII. Les plasmides recombinants sont établis chez E. coli puis on les fait pénétrer par électroporation dans L. lactis. Cette dernière est utilisée pour vérifier les structures des plasmides et les tailles des inserts. Les résultats sont présentés dans le tableau 4 suivant. On utilise les enzymes de restriction Hpal (site unique dans la partie du vecteur) et HindIII (site unique entre l'insert et le vecteur) pour analyser les intégrants.

**Tableau 4 :**

| Ipc à différentes localisations sur le chromosome de L. lactis | | |
|---|---|---|
| Taille de l'insert plasmidique (Kb) | | IPC moyenne ± SD |
| Groupe I : | | |
| pVE7025 | 1,29 | 3,0 ± 0,3 x 10⁻² |
| pVE7034 | 1,05 | 3,8 ± 0,5 x 10⁻³ |
| pVE7021 | 1,29 | 3,4 ± 2,6 x 10⁻³ |
| pVE7024 | 0,96 | 2,5 ± 1,3 x 10⁻³ |
| pVE7030 | 1,42 | 2,3 ± 0,8 x 10⁻³ |
| pVE7028 | 1,46 | 7,2 ± 0,7 x 10⁻⁴ |
| pVE7023 | 1,29 | 6,6 ± 3,9 x 10⁻⁴ |
| pVE7022 | 1,08 | 5,7 ± 0,3 x 10⁻⁴ |
| pVE7027 | 1,05 | 5,2 ± 1,3 x 10⁻⁴ |
| pVE7026 | 0,96 | 4,0 ± 0,5 x 10⁻⁴ |
| | | |

| Groupe II : | | |
|---|---|---|
| pVE7029 | 1,02 | 1,1 ± 0,4 x 10⁻⁵ |
| pVE7031 | 0,96 | 9,9 ± 3,9 x 10⁻⁶ |
| pVE7032 | 1,37 | 8,6 ± 5,0 x 10⁻⁷ |
| PVE7033 | 1,25 | 3,9 ± 0,9 x 10⁻⁷ |
| ipc : fréquence d'intégrations par cellule | | |

### c) clonage et délétion d'un fragment d'opéron ilv

Un fragment EcoRI de 3949 bp de l'opéron ilv de IL1403 (J. Bacteriol 174, 6580-6589 (1992) est cloné dans l'une ou l'autre orientation au site EcoRI de pG⁺host5 (pour donner pVE7009 et pVE7009R).

### Intégration par simple crossing-over (sco) dans le chromosome de L. lactis

Des souches de Lactococcus contenant les plasmides testés sont cultivées une nuit à 28° C en présence d'érythromycine, puis diluées 100 fois dans le même milieu et cultivées à 28° C pendant 2 heures à 2 heures 1/2 (phase exponentielle). Les cultures sont placées à 37,5° C pendant 3 heures afin de diminuer le nombre de copies de plasmides par cellule. Les échantillons sont ensuite dilués et étalés à 37° C sur milieu M17 Em afin de détecter les évènement d'intégration, et à 28° C sur milieu non sélectif pour déterminer le nombre de cellules viables. La fréquence d'intégration par cellule (ipc) est estimée comme étant le rapport des cellules Em^{r} à 37° C, sur le nombre de cellules viables à 28° C. Les intégrants isolés à 37° C sont maintenus dans un milieu M17 contenant Em, à 37,5° C pour un usage ultérieur.

### Intégration par double crossing-over (dco) dans le chromosome de L. lactis

Les plasmides pIL1263 et pIL1202, sont composés du vecteur Ts (pG⁺host4, Em^{r}) et respectivement des régions chromosomiques de 2,3 Kb ou 3,6 Kb, interrompues par le gène Tet de Tn1545 (Nucl. Acids Res., 14. 7047-7058, 1986). Une souche portant pIL1202 ou pIL1263 est cultivée une nuit à 37,5° C dans M17 avec Tet ou Em pour obtenir une population d'intégrants. La culture est ensuite diluée à 1/10⁵ dans du milieu M17 sans antibiotique et portée à 28° C pour stimuler la recombinaison par réplication plasmidique. Une culture de 12 heures ou plus à 28° C donne des fréquences maximales de remplacement du gène. Une culture d'une nuit à 28° C est étalée à différentes concentrations cellulaires à 37° C avec ou sans sélection par Tet. Les colonies dans lesquelles le remplacement du gène s'est produit ont un phénotype Tet^{r} et Em sensible (Em^{s}). L'ADN chromosomique est préparé selon des méthodes connues (Grüss et al, 1988)

L'ADN purifié est traité par des enzymes de restriction, séparé par électrophorèse en gel d'agarose et analysé par hybridation de Southern avec des sondes d'ADN pour détecter les recombinaisons homologues (Sambrook et al, 1989).

### Résultats

### 1) Intégration par simple crossing-over

Le clonage des fragments chromosomiques de L. lactis dans pG⁺host5 chez E. coli permet d'isoler 14 plasmides différents contenant chacun une insertion chromosomique distincte, de 0,9 Kb à 1,4 Kb. Ces plasmides établis dans IL1403 à 28° C, servent à mesurer les fréquences d'intégration dans le chromosome de L. lactis. La fréquence d'intégration par cellule est comprise entre 10⁻² et 10⁻⁷. L'ipc d'un vecteur pG⁺host5 sans insert chromosomique est compris entre 10⁻⁶ et 10⁻⁷. Les plasmides portant les inserts chromosomiques peuvent être classés en deux groupes en fonction de leur fréquence d'intégration. Dans le groupe 1, l'ipc varie entre 3.10⁻² et 4.10⁻⁴. Ces variations doivent être dues à la localisation ou à la nature de l'insert plutôt qu'à sa taille. Dans le groupe II, l'ipc du plasmide est comprise entre 10⁻⁵ et 3.10⁻⁷. Ceci est probablement dû à l'interruption d'un gène chromosomique essentiel, qui ne permet d'observer que les intégrations non homologues. Seuls deux de ces plasmides (pVE7028 et pVE7034) produisent des molécules de hauts poids moléculaires (HMW). L'analyse de l'ADN chromosomique obtenu à partir des souches intégrantes maintenues à 37° C, par restriction enzymatique et hybridation de Southern en utilisant le plasmide pG⁺host5 comme sonde, indique une intégration simple et multi-tandems dans le cas de huit plasmides et une intégration par copies multiples dans le cas de deux plasmides. La digestion par HindIII de l'ADN des intégrants confirme que l'intégration se produit par simple crossing-over. Chaque plasmide contient un seul site HindIII à la jonction vecteur-insert, et la digestion doit libérer une bande unique d'ADN de taille plasmidique. L'hybridation de Southern de l'ADN total non digéré ne révèle pas de plasmide libre dans aucun des plasmides du groupe 1, ce qui indique que la copie du plasmide est intégrée. Une analyse similaire des plasmides pVE7028 et pVE7034 confirme que ces plasmides sont également intégrés par simple crossing-over. L'utilisation d'Hpal, qui reconnaît un site unique à l'intérieur du vecteur, permet de déterminer que chaque plasmide est intégré à une position distincte.

Les quatre plasmides du groupe II (faible fréquence d'intégration), paraissent être intégrés au hasard, car la digestion par HindIII ne libère pas une bande monomérique de plasmide, et la digestion par Hpal de trois intégrants du même plasmide ne donne pas le même profil sur le gel. La carte de restriction du chromosome de L. lactis développé pour Smal et Apal permet de localiser les sites d'intégration des plasmides par simple crossing-over sur la carte chromosomique. Chaque intégrant est présent sur un segment différent. L'ensemble de ces résultats indique que les insertions chromosomiques sont positionnées de façon aléatoire sur le chromosome, excluant ainsi tout biais dans la procédure.

La fréquence d'intégration dépend de la longueur de l'homologie.

Un segment de 3,9 Kb de l'opéron ilv de IL1493 séquencé, est cloné dans pG⁺host5, et un ensemble de délétions du fragment est généré sur le même vecteur. Alors que des plasmides portant l'insert total de 3,9 Kb dans l'une des deux orientations (pVE7009 et pVE7009R) présentent une certaine instabilité structurelle dans L. lactis, les huit dérivés par délétion de pVE7009R sont stables. Ces clones sont utilisés pour étudier la relation entre la longueur de l'homologie et la fréquence d'intégration. Il existe une relation logarithmique entre la fréquence d'intégration et la longueur d'homologie pour des longueurs comprises entre 0,35 et 2,5 Kb. Pour des fragments de plus de 2,5 Kb, les fréquences de recombinaison paraissent atteindre un plateau, car les lpc des segments homologues de 2,5. 3,3 et 3,9 Kb ne sont pas significativement différents. Les facteurs autres que la longueur apparaissent aussi être importants. L'analyse par des enzymes de rectriction qui reconnaissent un site unique soit dans le vecteur, soit dans l'insert, soit dans la jonction vecteur-insert, confirme que l'intégration se produit par recombinaison homologue par simple crossing-over. Pour chaque plasmide utilisé, des intégrations multicopies du plasmide se produisent. Ces résultats montrent que pG⁺host fournit un moyen d'intégration efficace par simple crossing-over s' il porte des segments homologues aussi petits que 330 paires de bases.

### 2) intégration par double crossing-over

Dans le système par simple crossing-over (sco) décrit ci-dessus, le plasmide intégré est flanqué de séquences répétées. Aussi, quand les souches intégrantes, générées à 37° C, sont placées à 28° C, la réplication du plasmide stimule fortement un second événement de recombinaison. Cet évènement à pour conséquence une haute fréquence d'exision du réplicon, conduisant soit à la structure parentale, soit à la structure chromosomique dco (double crossing-over).

Une souche faiblement transformable de L. lactis, NCDO2118, qui est prototrophe pour les ammos acides ramifiés (Ilv, Leu, Val) et dans laquelle aucune modification génétique n'était réalisable jusqu'à présent, est utilisée. Deux dérivés de pG⁺host4 qui portent un segment chromosomique soit contigü, soit non contigü sont utilisés. pIL1263 contient un fragment chromosomique de 2,3 Kb, en amont de l'opéron ilv, interrompu par un segment d'ADN de 4 Kb contenant un marqueur de résistance à la tétracycline (Tet^{r}). La susbtitution du gène doit conduire à l'insertion du marqueur Tet^{r} dans le chromosome et laisser l'opéron ilv⁺ intact. Le plasmide pIL1202 contient des segments non contigüs de 1,1 Kb et 2,5 Kb, correspondant aux extrémités d'une région de 18,5 Kb, incluant l'opéron ilv, relié par le marqueur Tet^{r} de 4 Kb. Le remplacement du gène doit conduire à une délétion du chromosome de 14,9 Kb incluant l'opéron ilv et donnant un phénotype ilv⁻. Sélection du gène de remplacement : une souche contenant soit pIL1202 soit pIL1263 est cultivée dans des conditions indiquées ci-dessus, en utilisant Tet comme marqueur de sélection. Dans des expériences indépendantes avec pIL1263, 69% et 98% des colonies Tet^{r} étaient Em^{s} ; avec pIL1202 50% et 91% des colonies Tet^{r} sont Em^{s}. Dans les cultures de contrôle maintenues à 37° C pendant la même période, toutes les colonies Tet^{r} sont également Em^{r}. Ce résultat indique que la réplication dans les plasmides rc (rolling circle ou cercle roulant) stimule l'excision à partir du chromosome. Cinq colonies Em^{s} obtenues par intégration de pIL11202 sont cultivées sur milieu minimum dépourvu d'aminos acides ramifiés et sont ilv⁻, ce qui confirme que la recombinaison a eu lieu. La structure de la région chromosomique correspondante des cinq isolats Tet^{r} Em^{s} est étudiée par hybridation de Southern, qui confirme le remplacement du gène dans tous les cas.
Pas de sélection : un protocole identique a été utilisé sans sélection par Tet, afin de se placer dans le cas où le fragment chromosomique porté par le plasmide n'a pas de marqueur de sélection. Dans trois expériences utilisant pIL1263 (insertion de gène), 10% à 40% des colonies obtenues à 37° C sans sélection sont Tet^{r} Em^{s}, ce qui indique qu'un évènement de remplacement du gène a eu lieu. Pour pIL1202 (délétion du chromosome) 1% à 7% des colonies sont Tet^{r} Em^{s}, ce qut indique un remplacement du gène ; parmi les quatre colonies Tet^{r} Em^{s} testées, toutes sont ilv⁻. L'analyse de la structure chromosomique des quatre intégrants par dco de chaque type confirme que le remplacement se produit sans sélection d'un nouveau fragment inséré. Ces résultats démontrent la faisabilité du remplacement de gène sans laisser un marqueur antibiotique dans le chromosome. Ce protocole est donc adapté pour la modification chromosomique sans utilisation de marqueurs de sélection.

### Utilisation du pG⁺host dans d'autres bactéries gram positif

L'efficacité de recombinaison intermoléculaire dans douze localisations différentes du chromosome de B. subtilis a été déterminée en transformant des cellules compétantes par un plasmide non réplicatif (J. Bacteriol. 174, 5593-5587, 1992). Dans ces expériences, le segment homologue est invariant (insertion d'un fragment de pBR322). Les efficacités varient d'environ trois fois en fonction de la position de l'intégration. En utilisant le système pG⁺host sco plutôt que le vecteur non réplicatif, des expériences de recombinaison identique peuvent être effectuées sur les deux souches de B. subtilis avec les différences d'un ordre de trois dans les fréquences d'intégration. pG⁺host5 portant le fragment de 1,4 Kb de pBR322 est introduit dans les souches de B. subtilis d'intérêt. En utilisant la procédure sco décrite ci-dessus, la fréquence d'intégration varie entre 1,8 ± 0,6.10⁻³ et 6,1 ± 0,9.10⁻⁴. Les mêmes variations de trois fois sont observées entre les deux différentes localisations que celles obtenues avec le système non réplicatif. Ce résultat démontre l'efficacité du système.

### Exemple 3 : Modification génétique d'organismes non-transformables

Des organismes d'intérêt industriel tels que certains lactobacilles ne sont actuellement pas transformables, il est cependant possible d'y introduire des plasmides par conjugaison. Le locus de mobilisation oriT du plasmide pIP501 a été caractérisé. pIP501 est autotransférable dans certains de ces lactobacilles.

Ce fragment oriT a été cloné dans le plasmide Ts (plasmide Ts:oriT) ; sa capacité à être mobilisé en présence d'un plasmide "helper" (dérivé de pIP501) qui fournit les protéines de transfert en trans a été testée. Plusieurs croisements intra- ou inter-espèces ont été réalisés avec succès (Tableau 5) ; avec les exconjugants qui ne contiennent que le plasmide Ts:oriT, le procédé à venir d'intégration par recombinaison est applicable. On peut donc introduire des informations génétiques dans le chromosome de Lactobacillus bulgaricus espèce non-transformable de plus en plus utilisée par l'industrie laitière.

La finalité du procédé d'intégration par recombinaison est la modification des caractères génétiques de souches bactériennes. cela suppose que les propriétés à modififier soient caractérisés au niveau moléculaire. Le développement d'un système de transposition fonctionnel (combinaison du plasmide Ts d'un transposon) contituerait un apport appréciable en tant qu'outil génétique pour l'analyse de L. lactis.

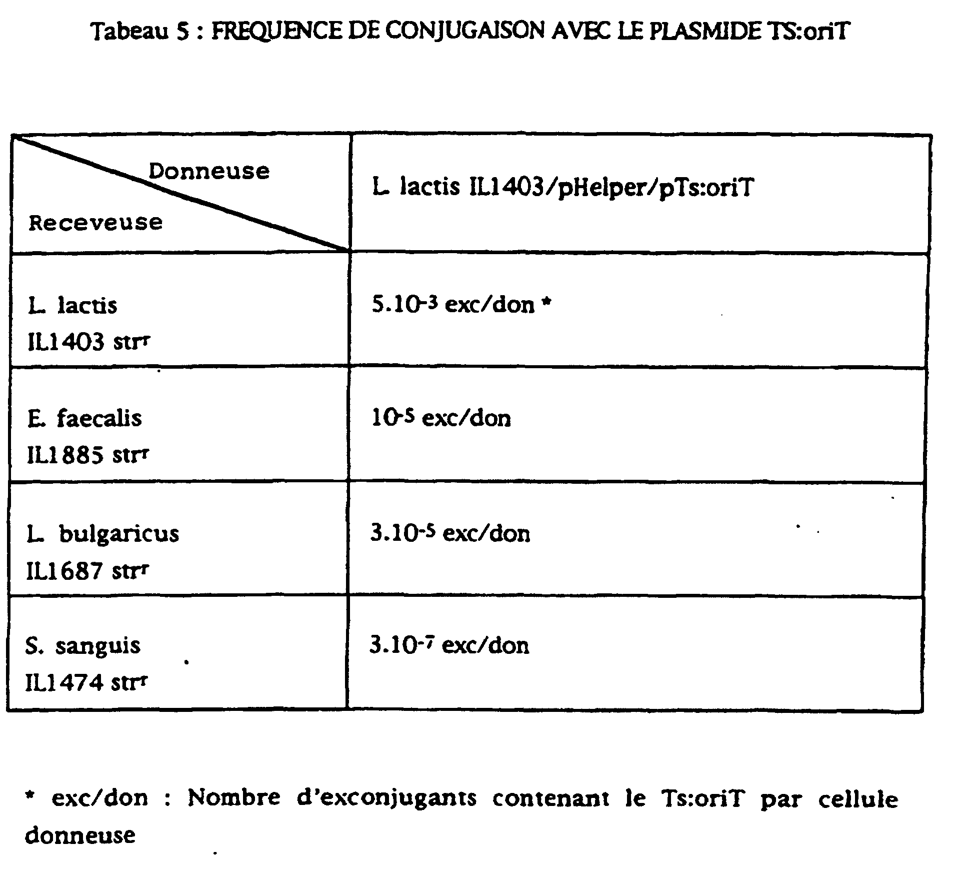

### Exemple 4 : utilisation d'un plasmide thermosensible comme vecteur d'un transposon

Une cassette de transposition Tn10 clonée dans un dérivé de pVE6004 est utilisée pour un test de transposition. Environ 1% des cellules sont Em^{r} à 37° C, ce qui indique que le transposon ou le plasmide est intégré dans le chromosome. L'intégration non spécifique du plasmide sans la cassette de transposition se produit à des fréquences inférieures à 10⁻⁷. La transposition est estimée par analyse de l'ADN digéré par HindIII à partir de huit colonies. HindIII a deux sites de restriction dans le plasmide, mais aucun dans l'unité transposable. L'ADN chromosomique est extrait de huit clones thermorésistants Em^{r} et digéré avec HindIII ; l'ADN traité est ensuite séparé par électrophorèse en gel d'agarose et hybridé avec un fragment d'ADN contenant le transposon Em^{r} comme sonde. Dans ces conditions, l'intégration du vecteur entier (c'est-à-dire sans transposition) conduirait à une bande d'hybridation de 1,3 Kb, qui n'est pas observée ici. Chaque échantillon chromosomique donne un profil unique quand le fragment d'ADN contenant le transposon est utilisé comme sonde. Aucune des bandes hybridées n'a une taille de 1,3 Kb, qui serait attendue si le plasmide entier était intégré dans un site du vecteur. En outre, on n'observe pas d'hybridation quand le plasmide vecteur Ts est utilisé comme sonde. Ces résultats indiquent que la transposition a lieu à différents sites et que l'ADN plasmidique ne s'intègre pas dans le chromosome avec le transposon. Le plasmide thermosensible peut donc être utilisé comme vecteur de livraison.

### LEGENDE DES FIGURES

### FIGURE 4 :

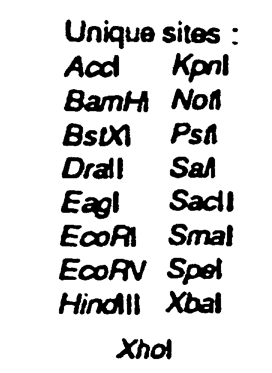

### FIGURE 6 :

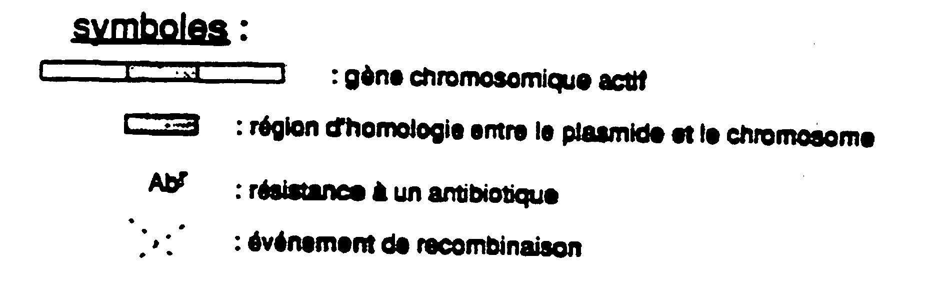
symboles :
 : gène chromosomique actif
 : région d'homologie entre le plasmide et le chromosome
Ab^r : résistance à un antibiotique
 : événement de recombinaison

### FIGURE 7A :

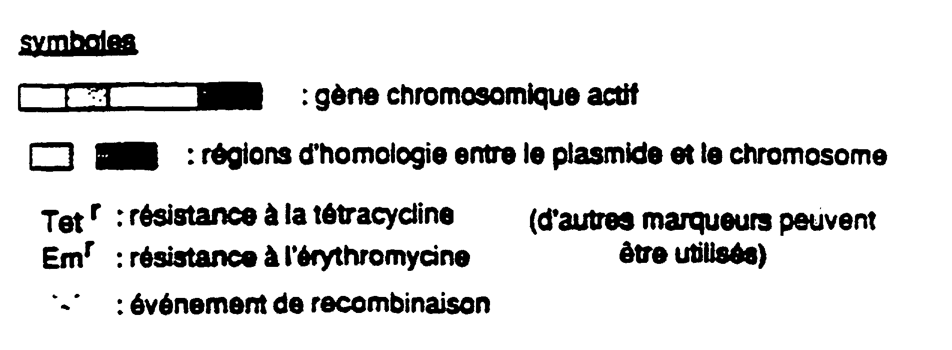
symboles

 : gène chromosomique actif

 : régions d'homologie entre le plasmide et le chromosome

Tet r : résistance à la tétracycline (d'autres marqueurs peuvent
Em r : résistance à l'érythromycine être utilisés)

 : événement de recombinaison

### FIGURE 7B :

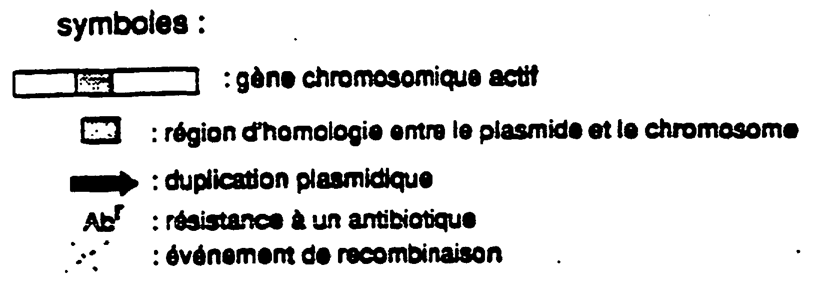
symboles :
 : gène chromosomique actif
 : région d'homologie entre le plasmide et le chromosome
 : duplication plasmidique
Ab^r : résistance à un antibiotique
 : événement de recombinaison

### REFERENCES

Chopin, A., MC. Chopin, A. Moillo-Batt, and P. Langella. 1984. Plasmid 11: 260-263.

Gasson, M.J. 1983. J. Bacteriol. **154**: 1-9.

Godon, J-J., C., Delorme, P., Renault and S. D. Ehrlich. 1992. Appl. Env. Microbiol. submitted.

Godon, JJ., MC. Chopin and S.D. Ehrlich. 1992. J. Bacteriol. **174** : 6580-6589.

Grüss, A., and S.D. Ehrlich. 1988. J. Bacteriol, **170** : 1183-1190

Hanahan, D. 1985. In DNA cloning: A practical approach Vol 1: 109-135. IRL Press Ed Glover.

Holo, H, and Nes, 1, F. 1989. Appl. Env. Microbiol. **55**: 3119-3123.

Kok. J., J.M.B. van der Vossen and G. Venema. 1984. Appl. Env. Microbiol. **48**: 726-731.

Langella, P., and Chopin, A. 1989. FEMS Microbiol. Lett. **89**: 301-306.

Leenhouts, K., J., B., Tolner, S., Bron, J., Kok, G., Venema and J., F.M.L. Seegers. 1991. Plasmid. **26**: 55-66.

Niaudet, B; Ehrlich, S, D. 1979. Plasmid. **2**: 48-58.

Petit, MA., Mesas, M., J., Noirot, P., and Ehrlich, S., D. 1992. Inducible Amplification in the Bacterial Chromosome, submitted.

Sambrook J., F Fritsh, and T., E. Mamatis.1989. Molecular cloning: a laboratory manual. Cold Spring Harbor Laboratory, Cold Spring Harbor, NY.

Thomas C.M. 1987. In Plasmids. A practical approach. IRL Press EDS Hardy.

## Revendications

1. Plasmide vecteur bactérien ayant une origine de réplication efficace dans E.coli et dans les bactéries gram positives choisies dans le groupe comprenant: Bacillus, Enteroccocus, Lactobacillus, Lactococcus, Streptococcus, Listeria, Pediococcus, Staphylococcus, Clostridia, Leuconostoc,
comportant au moins:
- un gène marqueur qui s'exprime dans une souche hôte bactérienne,
- un système de réplication efficace qui est thermosensible à partir d'une température compatible avec la viabilité de la souche hôte,
dont la température d'inhibition de réplication est inférieure ou égale à environ 37°C, et dont le système de réplication cercle roulant est celui porté par le plus grand fragment Cla1 du plasmide pWVO1, présentant au moins une mutation dans la région Tha1-Rsa1.

2. Plasmide vecteur selon la revendication 1, **caractérisé en ce qu'**il comporte en outre au moins une séquence d'ADN homologue avec une séquence d'ADN chromosomique, afin de permettre une recombinaison.

3. Plasmide vecteur selon la revendication 2, **caractérisé en ce que** le gène marqueur est situé de façon à être intégré dans le chromosome en cas de recombinaison.

4. Plasmide vecteur selon l'une des revendications 1 à 3, **caractérisé en ce que** le gène marqueur assure une résistance à un composé chimique ou est un gène permettant la complémentation d'une auxotrophie.

5. Plasmide vecteur selon l'une des revendications 1 à 4, **caractérisé en ce que** le système de réplication thermosensible est inhibé au-dessus de 35°C.

6. Plasmide vecteur selon l'une des revendications 1 à 5, **caractérisé en ce que** le plasmide comporte un locus de mobilisation permettant la conjugaison.

7. Plasmide vecteur selon l'une des revendications 1 à 6, **caractérisé en ce qu'**il comporte deux séquences identiques répétées, encadrant une séquence du plasmide.

8. Plasmide selon l'une des revendications 1 à 7, **caractérisé en ce qu'**il présente au moins une mutation dans la région correspondant à Rep A du plasmide pWVO1.

9. Plasmide selon la revendication 8, **caractérisé en ce que**, par rapport à la séquence de pWVO1, il présente au moins une mutation dans l'une des positions suivantes : 972, 977, 980, 987.

10. Plasmide selon l'une des revendications 8 et 9, **caractérisé en ce que** le fragment correspondant au système de réplication code pour une protéine présentant les mutations représentées sur la figure 3.

11. Plasmide selon l'une des revendications 1 à 10, **caractérisé en ce qu'**il comporte une des séquences représentées sur la figure 9, 10 ou 11, ou une séquence présentant au moins 80 % d'homologie avec ces séquences.

12. Plasmide selon l'une des revendications 8 à 11, **caractérisé en ce qu'**il est réplicatif à 28°C, et non-réplicatif à une température supérieure à environ 35°C.

13. Plasmide selon l'une des revendications 6 à 12, **caractérisé en ce que** le locus de mobilisation est le locus ori T, extrait d'un plasmide de bactérie à gram positif.

14. Plasmide selon l'une des revendications 6 à 13, **caractérisé en ce qu'**il comporte en outre un réplicon actif chez E. coli, qui en fait un plasmide navette gram-négatif, gram-positif.

15. Plasmide selon l'une des revendications 1 à 14, **caractérisé en ce qu'**il porte un transposon.

16. Plasmide selon l'une des revendications 1 à 15, **caractérisé en ce qu'**il comporte en outre un gène codant pour une protéine d'intérêt, sous le contrôle des éléments nécessaires à son expression.

17. Bactérie **caractérisée en ce qu'**elle contient un plasmide selon l'une des revendications 1 à 16, sous forme libre ou intégré dans son chromosome.

18. Procédé d'inactivation d'un gène présent dans le chromosome d'une bactérie, **caractérisé en ce que** :
a) on introduit dans la bactérie, par transformation, le plasmide selon l'une des revendications 1 à 5, 7 à 12, 14, 15 ou 16.
b) on cultive la bactérie sur milieu sélectif à une température inférieure à la température d'inhibition de l'origine de réplication,
c) on élève la température de culture à une température supérieure à ladite température d'inhibition,
d) on récupère les bactéries survivantes après plusieurs cycles de multiplication.

19. Procédé d'inactivation d'un gène dans une bactérie, **caractérisé en ce que** :
a) on introduit dans la bactérie, par conjugaison, un plasmide selon l'une des revendications 6 à 16,
b) on cultive la bactérie sur milieu sélectif à une température inférieure à la température d'inhibition de l'origine de réplication,
c) on élève la température de culture à une température supérieure à ladite température d'inhibition,
d) on récupère les bactéries survivantes après plusieurs cycles de multiplication.

20. Procédé d'introduction d'un gène hétérologue dans une bactérie, **caractérisé en ce que** :
a) on introduit dans la bactérie par transformation ou conjugaison un plasmide selon la revendication 16,
b) on cultive la bactérie sur milieu sélectif à une température inférieure à la température d'inhibition de l'origine de réplication,
c) on élève la température de culture à une température supérieure à ladite température d'inhibition,
d) on récupère les bactéries survivantes après plusieurs cycles de multiplication.

21. Procédé selon l'une des revendications 18 à 20, **caractérisé en ce que** les bactéries survivantes obtenues à l'issue de l'étape d), sont à nouveau mises en culture à une température inférieure à la température d'inhibition de l'origine de réplication, sur milieu non sélectif.

22. Procédé selon l'une des revendications 18 à 21, **caractérisé en ce que** l'étape b) est réalisée à une température d'environ 28°C.

## Patentansprüche

1. Bakterieller Plasmidvektor mit einem in E. coli und in grampositiven Bakterien, ausgewählt in der Gruppe, umfassend: Bacillus, Enterococcus, Lactobacillus, Lactococcus, Streptococcus, Listeria, Pediococcus, Staphylococcus, Clostridia, Leuconostoc, wirksamen Replikationsstartpunkt,
welcher wenigstens umfasst:
- ein Markergen, welches in einem bakteriellen Wirtsstamm exprimiert wird,
- ein wirksames Replikationssystem, welches ab einer Temperatur, welche mit der Lebensfähigkeit des Wirtsstamms verträglich ist, wärmesensitiv ist,
dessen Replikationsinhibitionstemperatur unter oder bei ungefähr 37°C liegt und dessen rollender Kreis-Replikationssystem jenes ist, welches sich auf dem größten Clal-Fragment des Plasmids pWVO1 befindet, welches wenigstens eine Mutation in der Tha1-Rsa1-Region aufweist.

2. Plasmidvektor nach Anspruch 1, **dadurch gekennzeichnet, dass** er außerdem wenigstens eine DNA-Sequenz umfasst, die zu einer chromosomalen DNA-Sequenz homolog ist, um eine Rekombination zu ermöglichen.

3. Plasmidvektor nach Anspruch 2, **dadurch gekennzeichnet, dass** das Markergen derart gelegen ist, dass es im Falle einer Rekombination in das Chromosom integriert wird.

4. Plasmidvektor nach einem der Ansprüche 1 bis 3, **dadurch gekennzeichnet, dass** das Markergen eine Resistenz gegen eine chemische Verbindung sicherstellt oder ein Gen ist, welches die Komplementation oder Ergänzung einer Auxotrophie ermöglicht.

5. Plasmidvektor nach einem der Ansprüche 1 bis 4, **dadurch gekennzeichnet, dass** das wärmesensitive Replikationssystem oberhalb von 35°C inhibiert wird.

6. Plasmidvektor nach einem der Ansprüche 1 bis 5, **dadurch gekennzeichnet, dass** das Plasmid einen Mobilisierungslocus, welcher die Konjugation ermöglicht, umfasst.

7. Plasmidvektor nach einem der Ansprüche 1 bis 6, **dadurch gekennzeichnet, dass** er zwei identische wiederholte Sequenzen umfasst, welche eine Sequenz des Plasmids einrahmen.

8. Plasmid nach einem der Ansprüche 1 bis 7, **dadurch gekennzeichnet, dass** es wenigstens eine Mutation in der Region, welche Rep A des Plasmids pWVO1 entspricht, aufweist.

9. Plasmid nach Anspruch 8, **dadurch gekennzeichnet, dass** es bezogen auf die Sequenz von pWVO1 wenigstens eine Mutation in einer der folgenden Positionen aufweist: 972, 977, 980, 987.

10. Plasmid nach einem der Ansprüche 8 und 9, **dadurch gekennzeichnet, dass** das dem Replikationssystem entsprechende Fragment ein Protein kodiert, welches die in der Figur 3 angegebenen Mutationen aufweist.

11. Plasmid nach einem der Ansprüche 1 bis 10, **dadurch gekennzeichnet, dass** es eine der in Figur 9, 10 oder 11 angegebenen Sequenzen oder eine Sequenz, welche wenigstens 80% Homologie zu diesen Sequenzen aufweist, umfasst.

12. Plasmid nach einem der Ansprüche 8 bis 11, **dadurch gekennzeichnet, dass** es bei 28°C replikativ ist und bei einer Temperatur über ungefähr 35°C nicht-replikativ ist.

13. Plasmid nach einem der Ansprüche 6 bis 12, **dadurch gekennzeichnet, dass** der Mobilisierungslocus der ori T-Locus, welcher aus einem Plasmid eines grampositiven Bakteriums extrahiert worden ist, ist.

14. Plasmid nach einem der Ansprüche 6 bis 13, **dadurch gekennzeichnet, dass** es außerdem ein in E. coli aktives Replikon umfasst, das tatsächlich ein gramnegativ/grampositiv-Shuttle-Plasmid ist.

15. Plasmid nach einem der Ansprüche 1 bis 14, **dadurch gekennzeichnet, dass** es ein Transposon trägt.

16. Plasmid nach einem der Ansprüche 1 bis 15, **dadurch gekennzeichnet, dass** es außerdem ein Gen, welches ein Protein von Interesse kodiert, unter der Kontrolle der für dessen Expression erforderlichen Elemente umfasst.

17. Bakterium, **dadurch gekennzeichnet, dass** es ein Plasmid nach einem der Ansprüche 1 bis 16 in freier oder in sein Chromosom integrierter Form enthält.

18. Verfahren zur Inaktivierung eines in dem Chromosom eines Bakteriums vorhandenen Gens, **dadurch gekennzeichnet, dass**:
a) man in das Bakterium durch Transformation das Plasmid nach einem der Ansprüche 1 bis 5, 7 bis 12, 14, 15 oder 16 einführt,
b) man das Bakterium auf selektivem Medium bei einer Temperatur unter der Inhibitionstemperatur des Replikationsstartpunkts kultiviert,
c) man die Kultivierungstemperatur auf eine Temperatur über der Inhibitionstemperatur erhöht,
d) man die nach mehreren Vermehrungszyklen überlebenden Bakterien gewinnt.

19. Verfahren zur Inaktivierung eines Gens in einem Bakterium, **dadurch gekennzeichnet, dass**:
a) man in das Bakterium durch Konjugation ein Plasmid nach einem der Ansprüche 6 bis 16 einführt,
b) man das Bakterium auf selektivem Medium bei einer Temperatur unter der Inhibitionstemperatur des Replikationsstartpunkts kultiviert,
c) man die Kultivierungstemperatur auf eine Temperatur über der Inhibitionstemperatur erhöht,
d) man die nach mehreren Vermehrungszyklen überlebenden Bakterien gewinnt.

20. Verfahren zur Einführung eines heterologen Gens in ein Bakterium, **dadurch gekennzeichnet, dass**:
a) man in das Bakterium durch Transformation oder Konjugation ein Plasmid nach Anspruch 16 einführt,
b) man das Bakterium auf selektivem Medium bei einer Temperatur unter der Inhibitionstemperatur des Replikationsstartpunkts kultiviert,
c) man die Kultivierungstemperatur auf eine Temperatur über der Inhibitionstemperatur erhöht,
d) man die nach mehreren Vermehrungszyklen überlebenden Bakterien gewinnt.

21. Verfahren nach einem der Ansprüche 18 bis 20, **dadurch gekennzeichnet, dass** die am Ende von Schritt d) erhaltenen überlebenden Bakterien erneut bei einer Temperatur unter der Inhibitionstemperatur des Replikationsstartpunkts auf nicht-selektivem Medium kultiviert werden.

22. Verfahren nach einem der Ansprüche 18 bis 21, **dadurch gekennzeichnet, dass** der Schritt b) bei einer Temperatur von ungefähr 28°C ausgeführt wird.

## Claims

1. Bacterial vector plasmid having an origin of replication which is effective in E. coli and in Gram-positive bacteria chosen from the group comprising: Bacillus, Enteroccocus, Lactobacillus, Lactococcus, Streptococcus, Listeria, Pediococcus, Staphylococcus, Clostridia, Leuconostoc,
containing at least:
- a marker gene which is expressed in a bacterial host strain,
- an effective replication system which is temperature-sensitive at and above a temperature compatible with the viability of the host strain,
the temperature of inhibition of replication of which is below or equal to approximately 37°C and the rolling circle replication system of which is that carried by the larger Cla1 fragment of plasmid pWV01, possessing at least one mutation in the Tha1-Rsa1 region.

2. Vector plasmid according to Claim 1, **characterized in that** it contains, in addition, at least one DNA sequence homologous with a chromosomal DNA sequence, so as to permit recombination.

3. Vector plasmid according to Claim 2, **characterized in that** the marker gene is located so as to be integrated in the chromosome in the case of recombination.

4. Vector plasmid according to one of Claims 1 to 3, **characterized in that** the marker gene provides for resistance to a chemical compound or is a gene permitting the complementation of an auxotrophy.

5. Vector plasmid according to one of Claims 1 to 4, **characterized in that** the temperature-sensitive replication system is inhibited above 35°C.

6. Vector plasmid according to one of Claims 1 to 5, **characterized in that** the plasmid contains a mobilization locus permitting conjugation.

7. Vector plasmid according to one of Claims 1 to 6, **characterized in that** it contains two identical repeat sequences flanking a sequence of the plasmid.

8. Plasmid according to one of Claims 1 to 7, **characterized in that** it possesses at least one mutation in the region corresponding to Rep A of plasmid pWVO1.

9. Plasmid according to Claim 8, **characterized in that**, relative to the sequence of pWV01, it possesses at least one mutation in one of the following positions: 972, 977, 980, 987.

10. Plasmid according to either of Claims 8 and 9, **characterized in that** the fragment corresponding to the replication system codes for a protein possessing the mutations shown in Figure 3.

11. Plasmid according to one of Claims 1 to 10, **characterized in that** it contains one of the sequences shown in Figure 9, 10 or 11, or a sequence possessing at least 80% homology with these sequences.

12. Plasmid according to one of Claims 8 to 11, **characterized in that** it is replicative at 28°C and non-replicative at a temperature above approximately 35°C.

13. Plasmid according to one of Claims 6 to 12, **characterized in that** the mobilization locus is the ori T locus, extracted from a plasmid of a Gram-positive bacterium.

14. Plasmid according to one of Claims 6 to 13, **characterized in that** it contains, in addition, a replicon which is active in E. coli, making it a Gram-negative, Gram-positive shuttle plasmid.

15. Plasmid according to one of Claims 1 to 14, **characterized in that** it carries a transposon.

16. Plasmid according to one of Claims 1 to 15, **characterized in that** it contains, in addition, a gene coding for a protein of interest, under the control of the elements needed for its expression.

17. Bacterium, **characterized in that** it contains a plasmid according to one of Claims 1 to 16, in free form or integrated in its chromosome.

18. Method for the inactivation of a gene present in the chromosome of a bacterium, **characterized in that**:
a) the plasmid according to one of Claims 1 to 5, 7 to 12, 14, 15 or 16 is introduced into the bacterium by transformation,
b) the bacterium is cultured on selective medium at a temperature below the temperature of inhibition of the origin of replication,
c) the culture temperature is raised to a temperature above said temperature of inhibition,
d) the surviving bacteria are recovered after several multiplication cycles.

19. Method for the inactivation of a gene in a bacterium, **characterized in that**:
a) a plasmid according to one of Claims 6 to 16 is introduced into the bacterium by conjugation,
b) the bacterium is cultured on selective medium at a temperature below the temperature of inhibition of the origin of replication,
c) the culture temperature is raised to a temperature above said temperature of inhibition,
d) the surviving bacteria are recovered after several multiplication cycles.

20. Method for the introduction of a heterologous gene into a bacterium, **characterized in that**:
a) a plasmid according to Claim 16 is introduced into the bacterium by transformation or conjugation,
b) the bacterium is cultured on selective medium at a temperature below the temperature of inhibition of the origin of replication,
c) the culture temperature is raised to a temperature above said temperature of inhibition,
d) the surviving bacteria are recovered after several multiplication cycles.

21. Method according to one of Claims 18 to 20, **characterized in that** the surviving bacteria obtained at the end of step d) are cultured again at a temperature below the temperature of inhibition of the origin of replication, on non-selective medium.

22. Method according to one of Claims 18 to 21, **characterized in that** step b) is carried out at a temperature of approximately 28°C.
